# FASCICULE DE BREVET EUROPEEN

(11) **EP 0 880 522 B1**
(45) Date de publication et mention de la délivrance du brevet: **19.09.2001**
(21) Numéro de dépôt: 97900236.7
(22) Date de dépôt: 06.01.1997
(51) Int. Cl.: C07D 487/04, C07D 233/90, A61K 31/495, C07F 9/6561, C07F 9/6506

(54) **DERIVES DE 5H,10H-IMIDAZO[1,2-a]INDENO[1,2-e]PYRAZINE-4-ONE ANTAGONISTES DES RECEPTEURS AMPA ET NMDA, LEUR PREPARATION, LEURS INTERMEDIAIRES ET LES MEDICAMENTS LES CONTENANT**
5H,10H-IMIDAZO[1,2-a]INDENO[1,2-e]PYRAZIN-4-ONE DERIVATE ANTAGONISTEN DER REZEPTOREN AMPA UND NMDA, IHRE HERSTELLUNG UND ZWISCHENPRODUKTE UND SIE ENTHALTENDE ARZNEIMITTEL
5H,10H-IMIDAZO[1,2-a]INDENO[1,2-e]PYRAZINE-4-ONE DERIVATIVES AMPA AND NMDA RECEPTORS ANTAGONISTS, PREPARATION THEREOF, INTERMEDIATES THEREOF AND DRUGS CONTAINING SAME

(30) Priorité: 10.01.1996 FR 9600192
(43) Date de publication de la demande: 02.12.1998
(73) Titulaire: Aventis Pharma S.A., 92160 Antony (FR)
(72) Inventeur: ALOUP, Jean-Claude, F-94290 Villeneuve-le-Roi (FR); BOUQUEREL, Jean, F-92700 Drancy (FR); DAMOUR, Dominique, F-94310 Orly (FR); HARDY, Jean-Claude, F-95800 Cergy-Saint-Christophe (FR); JIMONET, Patrick, F-78450 Villepreux (FR); MANFRE, Franco, F-94450 Limeil-Brevannes (FR); MIGNANI, Serge, F-92290 Châtenay-Malabry (FR); NEMECEK, Patrick, F-94230 Thiais (FR)
(86) Numéro de dépôt international: FR9700019
(87) Numéro de publication internationale: WO9725328

(56) Documents cités:
- WO-A-95/26349
- WO-A-96/31511

## Description

La présente invention concerne des composés de formule : 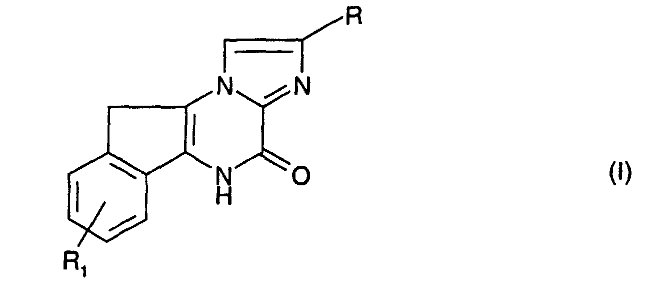 leurs isomères E et Z, leurs racémiques, énantiomères et diastéréoisomères, leurs sels, leur préparation, leurs intermédiaires et les médicaments les contenant.

Des dérivés d'indéno[1,2-e]pyrazin-4-one sont décrits dans les demandes de brevet WO94/07893, WO95/26349 et WO96/31511.

Dans la formule (I),
soit R représente un atome d'hydrogène ou un radical -COOH, -alk-COOH, -PO₃H₂, -CH₂-PO₃H₂, -CH=CH-COOH ou phényle substitué par un radical carboxy,
R₁ représente un radical -alk-CN, -alk-COOH, -alk-Het, -alk-PO₃H₂ ou
-alk-CO-NH-SO₂R₂,
R₂ représente un radical alkyle ou phényle,
alk représente un radical alkyle,
Het représente un cycle tétrazole-5-yle
étant entendu que lorsque R représente un atome d'hydrogène ou un radical
-COOH ou -PO₃H₂, R₁ ne peut pas représenter -alk-COOH,
soit R représente un radical -COOH et R₁ est en position 9 et représente un radical (4-phényl-1H-imidazol-2-yl-méthyl).

Sauf mention contraire, dans les définitions qui précédent et celles qui suivent, les radicaux alkyle et alcoxy contiennent 1 à 6 atomes de carbone en chaîne droite ou ramifiée.

De préférence, le substituant R₁ est en position -8 ou -9.

Les composés de formule (I) pour lesquels R représente un radical -CH=CH-COOH présentent des formes isomères (E et Z). Ces isomères et leurs mélanges font partie de l'invention.

Les racémiques, énantiomères et diastéréoisomères des composés de formule (I) pour lesquels R représente un radical -alk-COOH et/ou R1 représente un radical -alk-CN, -alk-COOH, -alk-Het, -alk-PO₃H₂ ou -alk-CO-NH-SO₂R₂ font également partie de l'invention.

Les composés de formule (I) pour lesquels R représente un atome d'hydrogène ou un radical -COOH, -alk-COOH, -PO₃H₂, -CH₂-PO₃H₂, -CH=CH-COOH ou phényle substitué par un radical carboxy, R₁ représente un radical -alk-CN, -alk-COOH, -alk-Het, -alk-PO₃H₂ ou -alk-CO-NH-SO₂R₂, R₂ représente un radical alkyle ou phényle peuvent être préparés par cyclisation, soit en présence d'acétate d'ammonium, soit en présence d'ammoniac, soit en présence d'acétate d'ammonium et d'ammoniac, d'un dérivé de formule : 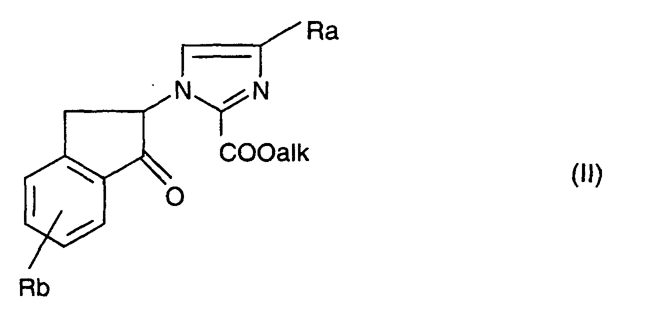 dans laquelle Ra représente un atome d'hydrogène ou un radical -COOalk, -alk-COOalk', -PO(Oalk)₂, -CH₂-PO(Oalk)₂, -CH=CH-COOalk' ou phényle substitué par un radical alcoxycarbonyle, Rb représente un radical -alk-CN, -alk-COOalk', -alk-PO(Oalk')₂, -alk-CO-NH-SO₂R₂ ou -alk-Het, alk et alk' représentent un radical alkyle, R₂ et Het ont les mêmes significations que dans la formule (I), suivie d'une hydrolyse.

Lorsque Het représente un radical tétrazolyle-5-yle, il est préférable d'utiliser un dérivé de formule (II) pour lequel Rb représente un radical -alk-tétrazole-5-yle dont le tétrazole est substitué en position -1 ou -2 par un radical benzyle puis de débenzyler le produit final.

Cette cyclisation s'effectue de préférence au sein d'un acide organique tel que l'acide acétique, à la température d'ébullition du milieu réactionnel éventuellement en présence d'ammoniac en solution dans un alcool aliphatique inférieur tel que le méthanol. L'hydrolyse des fonctions -COOalk, -PO(Oalk)₂ et la débenzylation s'effectuent par toute méthode connue permettant de passer d'un ester à l'acide correspondant ou d'un dialkylphosphonate à l'acide phosphonique correspondant ou de débenzyler sans modifier le reste de la molécule. De préférence, on opère au moyen d'un acide minéral tel que l'acide bromhydrique ou l'acide chlorhydrique, à une température de 100°C.

Les dérivés de formule (II) peuvent être obtenus par action d'une indanone de formule : 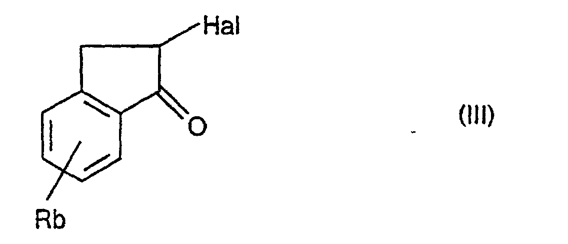 dans laquelle Rb a les mêmes significations que dans la formule (II) ou représente un radical -alk-Het' pour lequel Het' représente un radical tétrazole-5-yle substitué en position -1 ou -2 par un radical benzyle et Hal représente un atome d'halogène (chlore ou brome de préférence) sur un dérivé de formule : 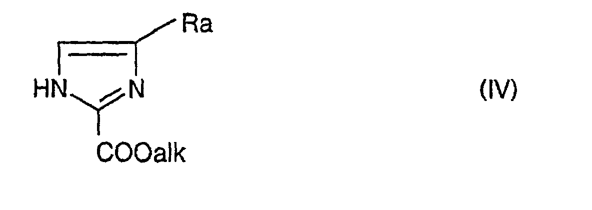 dans laquelle Ra a les mêmes significations que dans la formule (II) et alk représente un radical alkyle.

Cette réaction s'effectue généralement au sein d'un solvant inerte tel qu'un alcool (méthanol, éthanol par exemple), une cétone (acétone par exemple), un hydrocarbure aromatique (toluène par exemple), le diméthylformamide ou en absence de solvant, éventuellement en présence d'une base telle que l'hydrure de sodium ou le carbonate de potassium avec éventuellement un éther couronne tel que le 18C6, à une température comprise entre 20°C et la température d'ébullition du milieu réactionnel ou la fusion du milieu réactionnel.

Les dérivés de formule (II) pour lesquels Ra représente un radical -COOalk dans lequel alk n'est pas un radical tertbutyle et Rb représente un radical -alk-CO-NH-SO₂R₂ dans lequel R₂ a les mêmes significations que dans la formule (I) peuvent également être préparés par action d'un dérivé de formule (II) pour lequel Ra représente un radical -COOalk dans lequel alk n'est pas un radical tertbutyle et Rb représente un radical -alk-COOH sur un dérivé H₂N-SO₂R₂ dans lequel R₂ a les mêmes significations que dans la formule (I).

De préférence, on utilise un dérivé activé de l'acide au moyen de 1,1'-carbonyl-diimidazole. On opère généralement lorsque R₂ est un radical alkyle, au sein d'un solvant inerte tel que le tétrahydrofuranne, en présence d'une base organique azotée (1,8-diazabicyclo[5.4.0]undec-7-ène par exemple), à une température voisine de 20°C et lorsque R₂ est un radical phényle, au sein d'un solvant inerte tel que le diméthylformamide, en présence d'une base tel qu'un hydrure de métal alcalin (hydrure de sodium par exemple), à une température comprise entre 0 et 20°C.

Les dérivés de formule (II) pour lequel Ra représente un radical -COOalk dans lequel alk n'est pas un radical tertbutyle et Rb représente un radical -alk-COOH peuvent être obtenus par désestérification sélective du dérivé de formule (II) pour lequel Ra représente un radical -COOalk dans lequel alk n'est pas un radical tertbutyle et Rb représente un radical -alk-COOtertbutyle.

Cette réaction s'effectue au moyen d'un acide minéral tel que l'acide chlorhydrique, au sein d'un solvant inerte tel que le dioxanne à une température voisine de 20°C.

Les dérivés de formule (III) peuvent être obtenus par application ou adaptation des méthodes décrites par OLIVIER et coll., Bull. Soc. Chim. France, 3092 (1973), dans le brevet DE 2640358 et dans les exemples. De préférence, on halogène les indanones correspondantes au moyen d'un agent d'halogénation tel que le brome, le chlore, au sein d'un solvant inerte tel qu'un solvant chloré (chlorure de méthylène, chloroforme par exemple) ou l'acide acétique, à une température comprise entre -15°C et 20°C, ou un halogènure de cuivre, au sein du dioxanne, à une température voisine de 100°C ou par application ou adaptation des méthodes décrites par K. MORI, Agr. Biol. Chem., 27 (1), 22 (1963); J. CHAKRAVARTY, Indian J. Chem., 7 (3), 215 (1969), F. G. HOLLIMAN et coll., J. Chem. Soc., 9 (1960), D. MUKHOPADHYA et coll., J. Indian Chem. Soc., 47 (5), 450 (1970), dans les brevets DE 2640358, EP 346107 et dans les exemples.

'Les indanones correspondantes peuvent être obtenues par application ou adaptation des méthodes décrites dans les exemples. En particulier, les indanones substituées sur le noyau aromatique par un radical -alk-COOalk', -alk-CN ou -alk-Het, alk et alk' étant des radicaux alkyle peuvent être préparées selon le shéma réactionnel suivant : 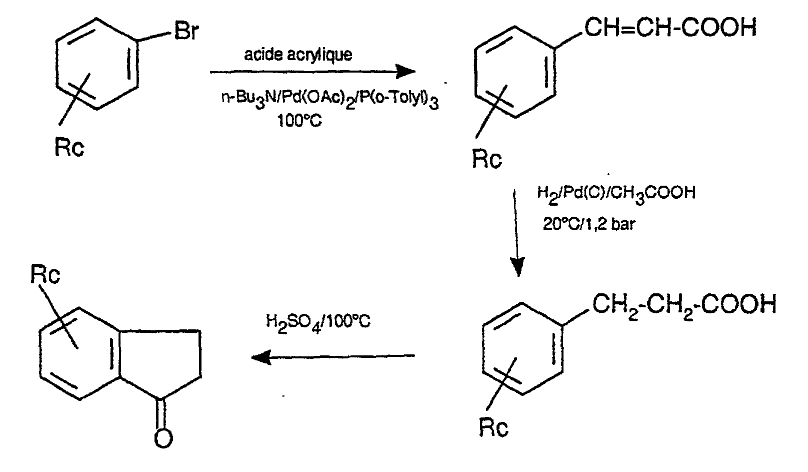 dans ces formules Rc représente un radical -alk-COOalk', -alk-CN ou -alk-Het, alk et alk' représentant des radicaux alkyle. Dans le cas où Rc représente un radical -alk-COOalk', le procédé comprend une étape finale supplémentaire d'estérification au moyen de chlorure d'oxalyle et d'un alcool aliphatique inférieur, au sein du dichlorométhane, à une température de 20°C. Les bromobenzènes substituées sur le noyau aromatique par un radical -alk-Het peuvent être obtenues par action d'un dérivé organométallique de l'hétérocycle (organolithien, organomagnésien par exemple) sur le bromobenzène substitué sur le noyau aromatique par un radical -alk-Br, au sein d'un éther ou du diméthylformamide, à une température de -70°C à 25°C. Les dérivés organométalliques des hétérocycles peuvent être obtenus par application ou adaptation des méthodes décrites par L. ESTEL et coll., J. Heterocyclic Chem., 26, 105 (1989); N.S. NARASIMHAN et coll., Synthesis, 957 (1983); A. TURCK et coll., Synthesis, 881 (1988); A.J. CLARKE et coll., Tetrahedron Lett, 27, 2373 (1974); A.R. KATRITZKY et coll., Org. Prep. Procedure Int., 20 (6), 585 (1988); N. FURUKAWA et coll., Tetrahedron Lett., 28 (47), 5845 (1987); V. SNIECKUS, Chem. Rev., 90, 879 (1990, L.J. BALDWIN et coll., J. Het. Chem., 22 (6), 1667 (1985), G. QUEGUINER et coll., Tetrahedron, 42 (8), 2253 (1986), G. QUEGUINER et coll., Tetrahedron, 51 (47), 13045 (1995) et M. ISHIKURA et coll., Heterocycle, 24 (10), 2793 (1986). Les bromobenzènes substitués par -alk-Br peuvent être obtenus par application ou adaptation des méthodes décrites par H. GILMAN, J. Org. Chem., 30, 325 (1965), C.H. DEPUY, J. Am. Chem. Soc., 79, 3710 (1957), S.A. GLOVER, Tetrahedron, 46 (20), 7247 (1990), J. OKADA, Chem. Pharm. Bull., 31 (9), 3074 (1983) et C.K. BRADSHER, J. Org. Chem., 46, 4600 (1981).

Les bromobenzènes substitués par -alk-CN peuvent être obtenus par application ou adaptation des méthodes décrites par PATAl, The chemistry of the cyano group, Wiley, New York, 1970.

Les bromobenzènes substitués par -alk-COOalk' peuvent être obtenus par application ou adaptation des méthodes décrites par LAROCK, Comprehensive Organic Transformations, VCH, New York, 1989.

Les indanones substituées sur le noyau aromatique par un radical -alk-Het pour lequel Het représente un radical tétrazole-5-yle dont le tétrazole est substitué en position -1 ou -2 par un radical benzyle peuvent être obtenues selon le shéma réactionnel suivant : 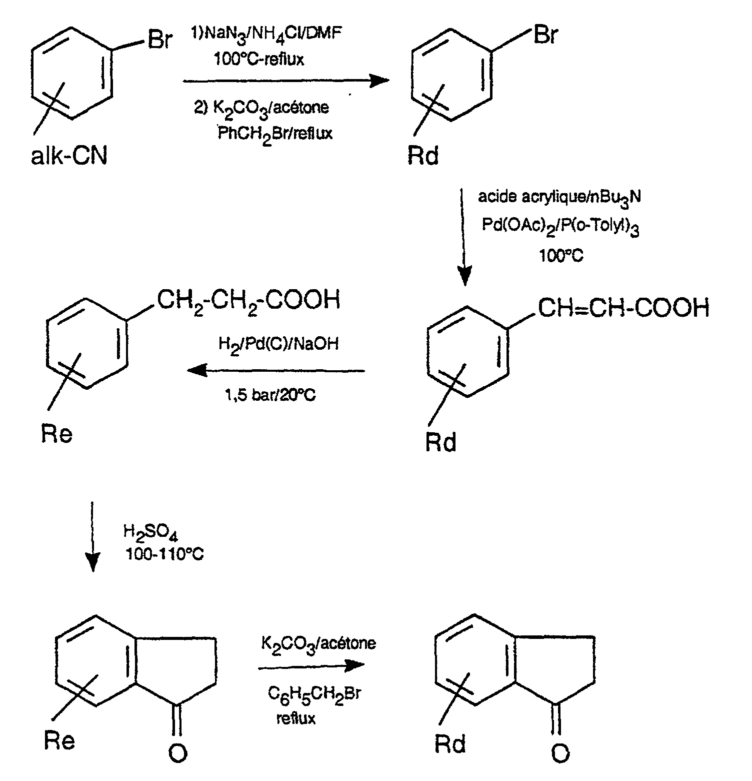 dans ces formules, Rd représente un radical -alk-tétrazol-5-yle dont le tétrazole est substitué en position -1 ou -2 par un radical benzyle et Re représente un radical -alk-tétrazol-5-yle.

Les indanones substituées sur le noyau aromatique par un radical -alk-PO(Oalk')₂ peuvent être obtenues selon le shéma réactionnel suivant : 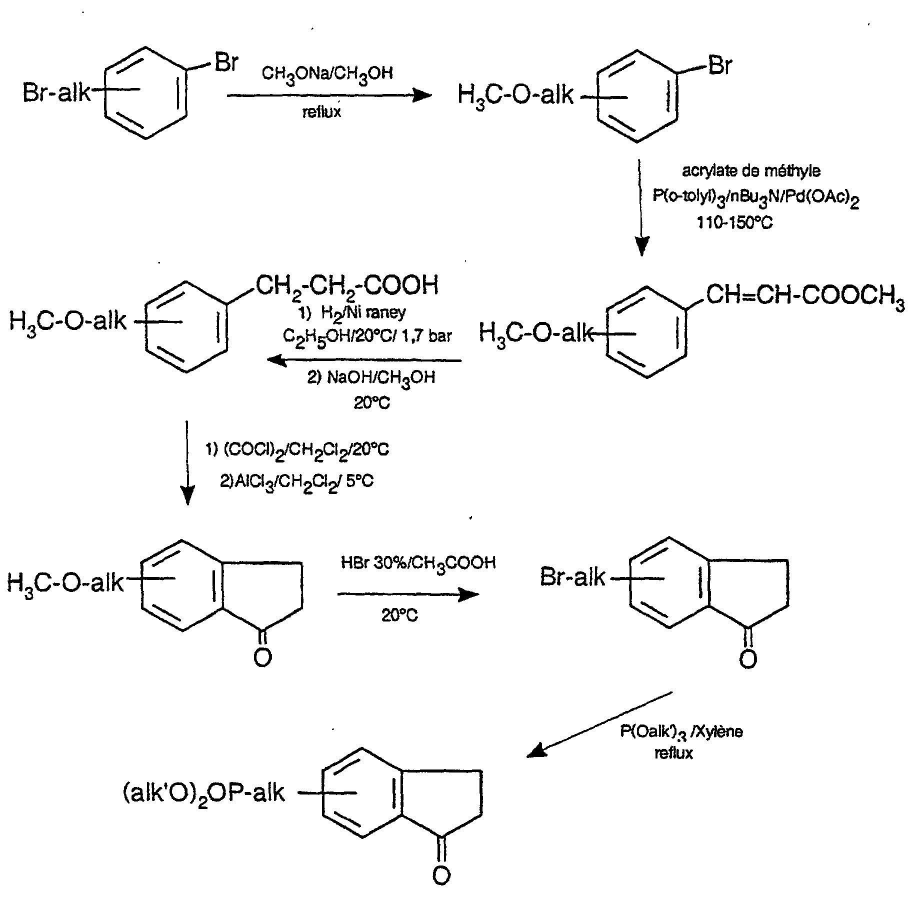 dans ces formules alk et alk' représentent des radicaux alkyle.

Les indanones substituées sur le noyau aromatique par un radical -alk-CO-NH-SO₂R₂ dans lequel alk représente un radical alkyle et R₂ a les mêmes significations que dans la formule (I) peuvent être obtenues par condensation d'une indanone substituée sur le noyau aromatique par un radical -alk-COlm pour lequel alk représente un radical alkyle et Im représente un radical imidazole avec un dérivé R₂-SO₂-NHNa pour lequel R₂ a les mêmes significations que dans la formule (I), à une température voisine de 0°C. L'indanone substituée sur le noyau aromatique par un radical -alk-COlm peut être obtenue par action d'une indanone substituée par un radical -alk-COOH sur le carbonylimidazole, au sein du tétrahydrofuranne, à une température voisine de 20°C. Le dérivé R₂-SO₂-NHNa peut être obtenu par action d'hydrure de sodium sur un dérivé de formule R₂-SO₂-NH₂, au sein du tétrahydrofuranne, à une température voisine de 20°C.

Les dérivés de formule (IV) pour lesquels Ra représente un atome d'hydrogène ou un radical -COOalk peuvent être obtenus par application ou adaptation des méthodes décrites par P.S. BRANCO et coll., Tetrahedron, 48 (30), 6335 (1992) et dans le brevet US 3600399.

Les dérivés de formule (IV) pour lesquels Ra représente un radical -PO(Oalk)₂ peuvent être obtenus par action d'un (hydroxyamino)iminoacétate d'alkyle sur un éthynylphosphonate de dialkyle, au sein du chloroforme, à une température comprise entre 20 et 50°C. Les (hydroxyamino)iminoacétates d'alkyle peuvent être obtenus par application ou adaptation de la méthode décrite par W.K. WARBURTON, J. Chem. Soc.(C), 1522 (1966) et les éthynylphosphonates de dialkyle peuvent être obtenus par application ou adaptation de la méthode décrite dans les exemples et par D.T. MONAGHAN et coll., Brain Res., 278, 138 (1983).

Les dérivés de formule (IV) pour lesquels Ra représente un radical -alk-COOalk', -CH₂-PO(Oalk)₂ ou phényle substitué par un radical alcoxycarbonyle sont nouveaux et font partie de l'invention ainsi que leur préparation. Ils peuvent être obtenus par action de H₂N-CH₂-CO-Rf sous forme de sel avec un acide minéral (chlorhydrate par exemple) dans lequel Rf représente un radical -alk-COOalk', -CH₂-PO(Oalk)₂ ou phényle substitué par un radical alcoxycarbonyle sur alkOOC-C(=NH)-Salk',BF₄H, dans lesquels alk et alk' représentent des radicaux alkyle, généralement au sein de l'acide acétique, en présence d'acétate de sodium, à la température d'ébullition du milieu réactionnel. Les dérivés H₂N-CH₂-CO-Rf sous forme de sel avec un acide minéral (chlorhydrate par exemple) pour lesquels Rf représente -alk-COOalk' peuvent être obtenus par application ou adaptation de la méthode décrite par D.E. ORR et coll., Chem. Ind. (London), 392 (1983). Les dérivés H₂N-CH₂-CO-Rf sous forme de sel avec un acide minéral (chlorhydrate par exemple) pour lesquels Rf représente -CH₂-PO(Oalk)₂ peuvent être obtenus par action de H₃C-PO(Oalk)₂ pour lequel alk représente un radical alkyle sur H₂N-alk-COOH dont la fonction amino est protégé par exemple par un radical tert-butoxycarbonyle et la fonction acide activée par exemple par le 1,1'-carbonyldiimidazole, en présence de butyllithium, au sein du tétrahydrofuranne à une température de -75°C puis libération de la fonction amine au moyen d'un acide minéral tel que l'acide chlorhydrique, au sein du dioxanne, à une température voisine de 20°C. Les dérivés H₂N-CH₂-CO-Rf pour lesquels Rf représente un phényle substitué par un radical alcoxycarbonyle peuvent être obtenus par application ou adaptation des méthodes décrites dans le brevet EP52442 ou par MINORU SUZUKI, J. Pharm. Soc. Japan, 72, 305 (1952). Les dérivés alkOOC-C(=NH)-Salk',BF₄H peuvent être obtenus par application ou adaptation de la méthode décrite par H. YAMANAKA et coll., Chem. Pharm. Bull., 31 (1), 4549 (1983).

Les dérivés de formule (IV) pour lesquels Ra représente un radical -CH=CH-COOalk' ou -alk(2C en chaîne droite)-COOalk' sont nouveaux et font partie de l'invention ainsi que leur préparation. Ils peuvent être obtenus selon le schéma réactionnel suivant : 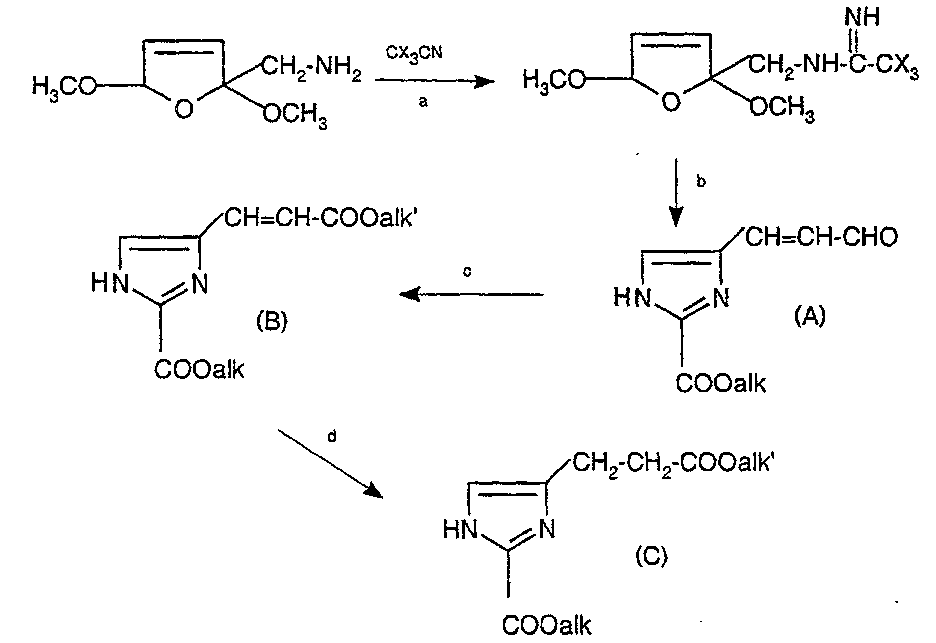 dans ces formules alk et aik' représentent des radicaux alkyle et X représente un atome d'halogène (chlore ou fluor de préférence) ou un radical alcoxy.

Dans l'étape a, la réaction s'effectue généralement au sein d'un solvant inerte tel qu'un éther (tétrahydrofuranne par exemple), à une température variant de -78°C à la température d'ébullition du milieu réactionnel.

Dans l'étape b, on fait réagir un acide minéral ou organique puis un alcool aliphatique (1-6C), Cette réaction s'effectue à une température variant de -20°C à la température d'ébullition du milieu réactionnel. Comme acide organique, on peut utiliser l'acide trifluoroacétique éventuellement au sein d'un solvant inerte tel qu'un solvant chloré (dichloroéthane par exemple) ou l'acide formique (96%). Comme acide minéral on peut utiliser l'acide chlorhydrique aqueux concentré ou l'acide sulfurique aqueux concentré.

Dans l'étape c, on fait réagir un alcool aliphatique (1-6C en chaine droite ou ramifiée) en présence d'acide persulfurique. Cette réaction s'effectue généralement à une température de 10 à 15°C. L'acide persulfurique peut être obtenu selon selon la méthode décrite par Nishihara, A. et Kubota, I. (J. Org. Chem., 33, 2525, (1968), procédure A).

Dans l'étape d, on hydrogène le dérivé (B). Cette hydrogénation s'effectue généralement au sein d'un solvant organique inerte tel que l'acétate d'éthyle ou l'acide acétique ou un mélange des 2 solvants, au moyen d'hydrogène, à une pression de 1 à 2 bar, en présence de charbon palladié, à une température voisine de 20°C.

Le dérivé (A) du shéma réactionnel précédent est nouveau et fait partie de l'invention ainsi que son procédé de préparation.

Les composés de formule (I) pour lesquels R représente un radical -alk-COOH dans lequel alk est un radical alkyle contenant 2 atomes de carbone en chaîne droite peuvent également être préparés par hydrogénation d'un composé de formule (I) correspondant pour lequel R représente un radical -CH=CH-COOH.

Cette réaction s'effectue par toute méthode permettant d'hydrogéner un dérivé acrylique sans toucher au reste de la molécule. En particulier, cette réaction s'effectue au moyen d'hydrogène, sous une pression comprise entre 3 et 8 bar, en présence d'un catalyseur d'hydrogénation tel que le charbon palladié, au sein d'une solution aqueuse d'hydroxyde de sodium, à une température comprise entre 30 et 50°C.

Les composés de formule (I) pour lesquels R représente un radical phényle substitué par un radical carboxy peuvent également être préparés par hydrolyse d'un dérivé correspondant pour lequel le radical phényle est substitué par un radical cyano.

Cette réaction s'effectue de préférence, au moyen d'un acide minéral tel que l'acide chlorhydrique, en milieu aqueux, à la température d'ébullition du milieu réactionnel.

Les composés de formule (1) peuvent être purifiés par les méthodes connues habituelles, par exemple par cristallisation, chromatographie ou extraction.

Les énantiomères des composés de formule (I) peuvent être obtenus par dédoublement des racémiques par exemple par chromatographie sur colonne chirale selon W.H. PIRCKLE et coll., asymetric synthesis, vol. 1, Academic Press (1983) ou par synthèse à partir des précurseurs chiraux.

Les isomères et diastéréoisomères des composés de formule (I) peuvent être séparés par les méthodes connues habituelles, par exemple par cristallisation ou chromatographie.

Les composés de formule (I) comportant un reste basique peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés sont des antagonistes du récepteur de l'acide α-amino-3-hydroxy-5-méthyl-4-isoxazolepropionique (AMPA), connu aussi sous le nom de récepteur du quisqualate.

Par ailleurs, les composés de formule (I) sont des antagonistes non compétitifs du récepteur N-méthyl-D-aspartate (NMDA) et, plus particulièrement, ce sont des ligands pour les sites modulateurs de la glycine du récepteur NMDA.

Ces composés sont donc utiles pour traiter ou prévenir toutes les ischémies (telles l'ischémie focale ou globale) consécutives à des accidents vasculaires cérébraux tels que le stroke thromboembolique et hémorragique, un arrêt cardiaque, une hypotension artérielle, une intervention chirurgicale cardiaque, vasculaire ou pulmonaire ou une hypoglycémie sévère. Ils sont également utiles dans le traitement des effets dus à une anoxie, qu'elle soit périnatale ou consécutive à une noyade, une haute pression ou à des lésions cérébro-spinales. Ces composés peuvent également être utilisés pour traiter ou prévenir l'évolution de maladies neurodégénératives, de la chorée d'HUNTINGTON, de la maladie d'ALZHEIMER et autres démences, de la sclérose latérale amyotrophique ou d'autres maladies du motoneurone, de l'atrophie olivo-pontocérébelleuse et de la maladie de PARKINSON. Ces composés peuvent aussi être utilisés vis-à-vis des manifestations épileptogènes et/ou convulsives, pour le traitement des traumatismes cérébraux ou spinaux, des traumatismes liés à la dégénérescence de l'oreille interne (R. PUJOL et coll., Neuroreport, 3, 299-302 (1992) ou de la rétine (J.L. MONSINGER et coll., Exp. Neurol., 113, 10-17 (1991), du tinnitus, de l'anxiété (KEHNE et coll., Eur. J. Pharmacol., 193, 283 (1991)), de la dépression (TRULLAS et coll.,Eur. J. Pharmacol., 185, 1 (1990)), de la schizophrénie (REYNOLDS, TIPS, 13, 116 (1992)), du syndrome de TOURETTE, des encéphalopathies hépatiques, des troubles du sommeil, des désordres du déficit attentionnel, des troubles des conditions hormonales (excès de la sécrétion de HG ou HL, sécrétion de corticostérone), en tant qu'analgésiques (DICKENSON et coll., Neurosc. Letters, 121, 263 (1991)), antiinflammatoires (SLUTA et coll., Neurosci. Letters, 149, 99-102 (1993)) antianorexiques (SORRELS et coll., Brain Res., 572, 265 (1992)), antimigraineux, antiémétiques et pour traiter les empoisonnements par des neurotoxines ou d'autres substances agonistes du récepteur NMDA ou AMPA, ainsi que les troubles neurologiques associés aux maladies virales telles que les méningites et encéphalites virales, le SIDA (LIPTON et coll., Neuron, 7, 111 (1991)), la rage, la rougeole et le tétanos (BAGETTA et coll., Br. J. Pharmacol., 101, 776 (1990)). Ces composés sont aussi utiles pour la prévention, la tolérance et la dépendance des symptômes d'abstinence aux drogues, à l'alcool et de l'inhibition de l'accoutumance et de la dépendance aux opiacés, barbituriques, amphétamine et benzodiazépines. Ils peuvent également être utilisés dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication chronique au plomb) et la déficience en sulfite oxydase.

L'affinité des composés de formule (I) vis-à-vis du récepteur AMPA a été déterminée en étudiant l'antagonisme de la fixation spécifique du [³H]-AMPA sur des membranes de cortex cérébral de rat (HONORE et coll., Neuroscience letters, 54, 27 (1985)). Le [³H]-AMPA est mis à incuber en présence de 0,2 mg de protéines à 4°C pendant 30 minutes dans du tampon KH₂PO₄ 10mM, KSCN 100mM, pH7,5. La fixation non spécifique est déterminée en présence de L-glutamate 1mM. La radioactivité liée est séparée par filtration sur filtres PHARMACIA (Printed Filtermate A). L'activité inhibitrice de ces produits est inférieure ou égale à 100 µM.

L'affinité des composés de formule (I) pour le site glycine lié au récepteur NMDA a été déterminée en étudiant l'antagonisme de la fixation spécifique du [³H]-DCKA sur des membranes de cortex cérébral de rat selon la méthode décrite par T. CANTON et coll., J. Pharm. Pharmacol., 44, 812 (1992). Le [³H]-DCKA (20nM) est mis à incuber en présence de 0,1 mg de protéines à 4°C pendant 30 minutes dans du tampon HEPES 50 mM, pH7,5. La fixation non spécifique est déterminée en présence de glycine 1mM. La radioactivité liée est séparée par filtration sur filtres Whatman GF/B. L'activité inhibitrice de ces produits est inférieure ou égale à 100 µM.

Les composés de formule (I) présentent une toxicité faible. Leur DL50 est supérieure à 50 mg/kg par voie IP chez la souris.

Les exemples suivants illustrent l'invention.

### PREPARATION DES INTERMEDIAIRES DE FORMULE (IV)

### EXEMPLE A : 4-(diéthoxyphosphoryl)-imidazole-2-carboxylate d'éthyle

On refroidit à une température voisine de 10°C une solution de 1,2 g d'(hydroxyamino)iminoacétate d'éthyle dans 20 ml de chloroforme et 1,4 ml de triéthylamine, et on ajoute goutte à goutte une solution de 1,54 g d'éthynylphosphonate de diéthyle dans 5 ml de chloroforme. Le milieu réactionnel est agité pendant la nuit à une température voisine de 20°C, additionné de 0,15 g d'éthynylphosphonate de diéthyle et chauffé pendant 1 heure à une température voisine de 50°C. Le mélange réactionnel est additionné de 50 ml de dichlorométhane et lavé avec 3x40 ml de solution saturée de chlorure de sodium. La phase organique est évaporée au rotavapor et le résidu d'évaporation est additionné de 40 ml d'éther éthylique et filtré. Le filtrat est évaporé au rotavapor pour donner une huile jaune (2,4 g). A cette huile on ajoute 20 ml de xylène et l'on chauffe à reflux pendant 20 heures. La phase liquide est décantée et évaporée au rotavapor. Le résidu d'évaporation est purifié par chromatographie sur colonne de silice en éluant avec de l'acétate d'éthyle. On obtient 0,5 g 4-(diéthoxyphosphoryl)-imidazole-2-carboxylate d'éthyle sous forme d'huile jaune [Spectre de masse (impact électronique) m/z 276 (M)^{+.}, 247 (276-C₂H₅)⁺, 231 (276-C₂H₅O)⁺, 204 (C₆H₉N₂O₄P)^{+.}, 157 (C₄H₂N₂O₃P)⁺].

L'(hydroxyamino)iminoacétate d'éthyle peut être synthétisé comme décrit par W.K. WARBURTON, J. Chem. Soc. (C), 1522 (1966).

L'éthynylphosphonate de diéthyle peut être synthétisé comme décrit par D.T. MONAGHAN et coll., Brain Res., 278, 138 (1983).

### EXEMPLE B : 4-(éthoxycarbonylméthyl)-imidazole-2-carboxylate d'éthyle

On chauffe pendant 3 heures à une température voisine de 95°C un mélange de 2,5 g d'(éthylthio)iminoacétate d'éthyle tétrafluoroborate, 110 ml d'acide acétique, 1,8 g de 4-aminoacétoacétate d'éthyle chlorhydrate et 1,64 g d'acétate de sodium. Le mélange réactionnel est filtré et l'insoluble est rincé avec 3x5 ml d'acide acétique. Le filtrat est évaporé au rotavapor et le résidu d'évaporation est additionné de 75 ml de dichlorométhane. La solution organique est séchée sur sulfate de sodium, filtrée et évaporée au rotavapor. Le résidu d'évaporation (1,86 g) est purifié par chromatographie sur colonne de silice en éluant avec de l'acétate d'éthyle. On obtient 1,5 g de 4-(éthoxycarbonylméthyl)-imidazole-2-carboxylate d'éthyle sous forme de solide jaune rouge fondant à 88°C.

L'(éthylthio)iminoacétate d'éthyle tétrafluoroborate peut être synthétisé comme décrit par H. YAMANAKA et coll., Chem. Pharm. Bull., 31(1), 4549 (1983).

Le 4-aminoacétoacétate d'éthyle chlorhydrate peut être synthétisé comme décrit par D.E. ORR et A.J. MIAH, Chem. Ind. (London), 392 (1983).

### EXEMPLE C : 2-éthoxycarbonylimidazole-4-méthylphosphonate de diéthyle

A une solution de 1,33 g de thiooxamate d'éthyle dans 50 ml de chlorure de méthylène on ajoute goutte à goutte à 20°C en 15 minutes une solution de 2,85 g de tétrafluoroborate de triéthyloxonium dans 10 ml de chlorure de méthylène. Après 16 heures d'agitation à la même température, la solution est concentrée à sec sous pression réduite. Le produit obtenu est dissous dans 10 mi d'acide acétique et on ajoute successivement une solution de 2,7 g de chlorhydrate de glycylméthylphosphonate de diéthyle dans 10 ml d'acide acétique et 1,64 g d'acétate de sodium. Le mélange est agité pendant 3 heures à une température voisine de 95°C et, après refroidissement à 20°C, l'insoluble apparu est séparé par filtration et lavé 2 fois avec 30 ml au total d'acide acétique. Le filtrat et le lavage sont réunis et concentrés à sec sous pression réduite. Le produit obtenu est chromatographié sur gel de silice neutre en éluant avec de l'acétate d'éthyle puis avec un mélange d'acétate d'éthyle et de méthanol (90-10 en volumes). Les fractions contenant le produit attendu sont réunies et concentrées à sec sous pression réduite et on obtient ainsi 2 g de 2-éthoxycarbonylimidazole-4-méthylphosphonate de diéthyle sous forme d'une huile rouge [RMN Spectre ¹H dans CDCl₃, T=300K, δ en ppm (250 MHz): 1,50 (6H, t, J=6Hz, 2 CH₃), 1,60 (3H, t, J=6Hz, CH₃), 3,50 (2H, d, J=20Hz, PCH₂), 3,27 et 3,78 (1H chacun, m, CH₂), 3,90 (2H, s, CH₂CO), 4,30 (4H, q, J=6Hz, P(OCH₂-)₂), 4,60 (2H, q, J=6Hz, OCH₂), 7,40 (1H, s, CH imidazole.), 12,0 (1H, s, NH)].

Le chlorhydrate de glycylméthylphosphonate de diéthyle peut être préparé de la manière suivante : on ajoute goutte à goutte en 10 minutes à 10°C 11 ml d'une solution 8N de dioxanne chlorhydrique à une solution de 3,41 g de N-t-butoxycarbonyl glycylméthylphosphonate de diéthyle dans 30 ml de dioxanne. Le mélange est agité pendant 3 heures à la même température puis concentré à sec sous pression réduite à 40°C. Le produit obtenu est mis en suspension 3 fois dans 225 ml au total d'éther éthylique anhydre, et séché après élimination du solvant surnageant. On obtient ainsi 2,7 g de chlorhydrate de glycylméthylphosphonate de diéthyle sous forme d'un produit gommeux [RMN Spectre ¹H dans DMSO-d6, T=300K, δ en ppm (250 MHz): 1,25 (6H, t, J=6Hz, CH₃), 3,52 (2H, d, J=20Hz, PCH₂), 4,00 (2H, s, NCH₂CO), 4,10 (4H, q, J=6Hz, P(OCH₂-)₂), 8,55 (1H, s, NH₂)].

Le N-t-butoxycarbonyl glycylméthylphosphonate de diéthyle peut être préparé de la manière suivante : à une solution de 8,75 g de N-t-butoxycarbonyl glycine dans 200 ml de tétrahydrofuranne anhydre on ajoute en 10 minutes à 20°C 8,1 g de 1,1'-carbonyldiimidazole. Le mélange est agité pendant 2 heures à la même température et on obtient ainsi une solution A. Sous couverture d'argon à une solution de 45,6 g de méthylphosphonate de diéthyle dans 400 ml de tétrahydrofuranne anhydre on ajoute en 45 minutes à -75°C 200 ml d'une solution 1,6 M de n-butyllithium dans l'hexane. Le mélange est agité pendant 75 minutes à la même température et on obtient ainsi une suspension blanche B. Sous couverture d'argon la solution A est ensuite ajoutée goutte à goutte en 40 minutes à la suspension B maintenue à -75°C. Le mélange est agité pendant 1 heure à la même température et pendant 1 heure à -30°C, additionné de 1,75 ml d'acide acétique, versé sur 1 I d'une solution aqueuse saturée d'hydrogénocarbonate de sodium et extrait 3 fois avec 2100 ml au total d'acétate d'éthyle. Les extraits organiques sont réunis, séchés sur du sulfate de sodium anhydre, filtrés et concentrés à sec sous pression réduite à 40°C. Le produit obtenu (23,6 g) est chromatographié sur gel de silice neutre en éluant avec de l'acétate d'éthyle. Les fractions contenant le produit attendu sont réunies et concentrées à sec sous pression réduite et on obtient ainsi 6,7 g de N-t-butoxycarbonyl glycylméthylphosphonate de diéthyle sous forme d'une huile incolore [RMN Spectre ¹H dans DMSO-d6, T=300K, δ en ppm (200 MHz): 1,25 (6H, t, J=6Hz, CH₃), 1,40 (9H, s, C(CH₃)₃), 3,27 (2H, d, J=20Hz, PCH₂), 3,92 (2H, d, J=6Hz, NCH₂CO), 4,10 (4H, q, J=6Hz, P(OCH₂-)₂), 7,05 (1H, t, J=6Hz, NH)].

### EXEMPLE D : 4(5)-(2-éthoxycarbonyl-éthyl)-1H-imidazole-2-carboxylate d'éthyle

Une solution de 4,2 g de (E)-4(5)-(2-éthoxycarbonyl-vinyl)-1H-imidazole-2-carboxylate d'éthyle dans 200 ml d'acétate d'éthyle et 2 ml d'acide acétique est hydrogénée en présence de 1 g de charbon palladié à 10% pendant 2 heures à une température voisine de 20°C et sous une pression voisine de 1,5 bar. Le mélange réactionnel est filtré et évaporé au rotavapor. On obtient 3,9 g de 4(5)-(2-éthoxycarbonyl-éthyl)-1H-imidazole-2-carboxylate d'éthyle sous forme de solide blanc fondant à 87°C [RMN Spectre ¹H dans CDCI3, T=300K, δ en ppm (250 Mhz) : 1,16 (3H, t, J=6Hz, CH₃), 1,30 (3H, t, J=6Hz, CH₃), 2,65 (2H, t, J=6Hz, CH₂), 2,81 (2H, t, J=6Hz, CH₂), 4,06 (2H, q, J=6Hz, OCH₂),), 4,29 (2H, q, J=6Hz, OCH₂), 7,02 (1H, s, CH imidazole)].

### EXEMPLE E : (E)-4(5)-(2-éthoxycarbonyl-vinyl)-1 H-imidazole-2-carboxylate d'éthyle

A une solution de 9,7 g de (E)-4(5)-(3-oxo-propényl)-1H-imidazole-2-carboxylate d'éthyle dans 500 ml d'éthanol refroidie entre 10 et 15°C on ajoute, goutte à goutte, 260 g (0,05 mol) d'acide persulfurique fraîchement préparé selon la méthode décrite par Nishihara, A. et Kubota, I. (J. Org.

Chem., 33, 2525, (1968), procédure A). On poursuit l'agitation pendant 1 heure et 30 minutes à cette température. Le milieu réactionnel est ensuite versé sur de la glace, neutralisé par une solution de carbonate de sodium et extrait par 2×750 ml d'acétate d'éthyle. La phase organique est séchée sur sulfate de magnésium et évaporée au rotavapor. On obtient 8,2 g de (E)-4(5)-(2-éthoxycarbonyl-vinyl)-1 H-imidazole-2-carboxylate d'éthyle sous forme de solide jaune fondant à 161°C [RMN Spectre ¹H dans CDCl₃ + AcOH, T=300K, δ en ppm (250 Mhz) : 1,27 (3H, t, J=6Hz, CH₃), 1,35 (3H, t, J=6Hz, CH₃), 4,20 (2H, q, J=6Hz, OCH₂), 4,39 (2H, q, J=6Hz, OCH₂), 6,55 (1H, d, J=16Hz, CH éthylénique), 7,40 (1H, s, CH imidazole), 7,55 (1H, d, J=16Hz, CH éthylénique)].

### EXEMPLE F : (E)-4(5)-(3-oxo-propényl)-1H-imidazole-2-carboxylate d'éthyle

Sous couverture d'argon, à 80 ml d'acide trifluoroacétique agité et refroidi à -15°C est ajoutée goutte à goutte une solution de 80 g de (+/-) (2,5-dihydro-2,5-diméthoxy-furane-2-ylméthyl)-(1-imino-2,2,2-trichloro-éthyl)-amine dans 80 ml de dichlorométhane. On poursuit l'agitation pendant 22 heures à une température voisine de 20°C. On ajoute alors 250 ml d'éthanol et chauffe à reflux. Le dichlorométhane est éliminé par distillation, puis le chauffage à reflux est maintenu pendant 3 heures et 30 minutes. Après refroidissement le milieu réactionnel est neutralisé par une solution d'hydrogénocarbonate de sodium et extrait par 3×500 ml d'acétate d'éthyle. La phase organique est séchée sur sulfate de magnésium et évaporée au rotavapor. Le résidu d'évaporation est purifié par filtration sur silice avec de l'acétate d'éthyle, puis trituration dans l'éther diéthylique. On obtient 9 g de (E)- 4(5)-(3-oxopropényl)-1 H-imidazole-2-carboxylate d'éthyle sous forme de solide jaune fondant à 151°C [RMN Spectre ¹H dans DMSO, T=300K, δ en ppm (300 Mhz) : 1,35 (3H, t, J=6Hz, CH₃), 4,39 (2H, q, J=6Hz, OCH₂), 6,55 (1H, s large, CH éthylénique), 7,45 (1H, d, J=16Hz, CH éthylénique), 7,75 (1H, s, CH imidazole)].

La (+/-)- (2,5-dihydro-2,5-diméthoxy-furane-2-ylméthyl)-(1-imino-2,2,2-trichloro-éthyl)-amine peut être préparée de la manière suivante : sous couverture d'argon, à une solution agitée et refroidie à une température voisine de -70°C de 45,4 g de 2,2,2-trichloroacétonitrile dans 80 ml de tétrahydrofuranne on ajoute, goutte à goutte, 50 g de (+/-)-2,5-dihydro-2,5-diméthoxyfurfurylamine commerciale. On poursuit l'agitation pendant 2 heures à une température voisine de 20°C. Le milieu réactionnel est additionné de 300 ml d'acétate d'éthyle et lavé par 2×100 ml de solution de solution saline. La phase organique est séchée sur sulfate de magnésium et évaporée au rotavapor. On obtient 81,5 g de (+/-)-(2,5-dihydro-2,5-diméthoxy-furane-2-ylméthyl)-(1-imino 2,2,2-trichloro-éthyl)-amine sous forme d'huile incolore visqueuse [RMN Spectre ¹H dans CDCI3, T=300K, δ en ppm (200 Mhz) : Mélange 50/50 des diastéréoisomères : 3,05 et 3,10 (3H, s, CH₃), 3,35 (3H, s, CH₃), entre 3,2 et 3,8 (2H, m, NCH₂), 5,35 et 5,65 (1H, s, OCH), entre 5,70 et 6,10 (2H, m, 2 CH éthylénique), entre 7,0 et 7,20 (1H, s, NH)].

### EXEMPLE G : 4(5)-(4-carbéthoxyphényl)-1H-lmldazole-2-carboxylate d'éthyle

A une solution de 1,33 g de thiooxamate d'éthyle dans 50 ml de chlorure de méthylène on ajoute goutte à goutte à 20°C en 10 minutes une solution de 2,85 g de tétrafluoborate de triéthyloxonium dans 10 ml de chlorure de méthylène. Le mélange est agité pendant 16 heures à une température voisine de 20°C puis concentré à sec sous pression réduite (15 mm Hg; 2 kPa) à 40°C. Le résidu (3,4 g) est dissous dans 20 ml d'acide acétique et on ajoute 2,43 g de chlorhydrate de 4-glycylbenzoate d'éthyle puis 1,64 g d'acétate de sodium anhydre. Le mélange est agité pendant 3 heures à 95°C puis concentré à sec sous pression réduite (15 mm Hg; 2 kPa) à 60°C. Le produit obtenu est additionné de 50 ml de chlorure de méthylène et de 50 ml d'eau distillée et, après décantation, la solution aqueuse est extraite 3 fois avec 90 ml au total de chlorure de méthylène. Les extraits organiques sont réunis, séchés sur du sulfate de sodium anhydre et concentrés à sec sous pression réduite (15 mm Hg; 2 kpa) à 40°C. Le résidu est chromatographié sur gel de silice neutre en éluant avec de l'acétate d'éthyle. On obtient ainsi 2,1 g de 4(5)-(4-carbéthoxyphényl)-1H-imidazole-2-carboxylate d'éthyle fondant à 174°C.

Le chlorhydrate de 4-glycylbenzoate d'éthyle peut être préparé de la manière suivante : 11,2 ml d'une solution aqueuse 10N d'acide chlorhydrique sont ajoutés à 120 ml d'éthanol absolu. A 84 ml de la solution ainsi obtenue, on ajoute 8,8 g de 4-(N,N-diformylglycyl)benzoate d'éthyle. Le mélange est agité pendant 16 heures à une température voisine de 20°C et l'insoluble apparu est séparé par filtration, lavé 3 fois avec 90 ml au total d'éther éthylique et séché sous pression réduite. On obtient ainsi 4,1 g de chlorhydrate de 4-glycylbenzoate d'éthyle sous forme d'une poudre blanche [Spectre ¹H dans DMSO-d6, T=300K, δ en ppm (300 MHz): 1,30 (3H, t, J=6Hz, CH₃), 4,35 (2H, q, J=6Hz, OCH₂), 4,60 (2H, s, COCH₂N), 8,10 (4H, m, PhCO₂H), 8,70 (3H, s, NH₂,HCl)].

Le 4-(N,N-diformylglycyl)benzoate d'éthyle peut être préparé de la manière suivante : à une solution de 13,55 g de 4-bromoacétylbenzoate d'éthyle dans 125 ml d'acétonitrile on ajoute 5,2 g de diformylamidure de sodium et le mélange est agité pendant 3 heures et 30 minutes à 80°C puis pendant 16 heures à une température voisine de 20°C. L'insoluble apparu est éliminé par filtration et le filtrat est concentré à sec sous pression réduite (15 mm Hg; 2 kPa) à 40°C. Le produit obtenu est mis 2 fois en suspension dans 150 ml au total d'éthanol et, après filtration et séchage (0,5 mm Hg; 0,07 kPa) à 40°C, on obtient 8,8 g de 4-(N,N-diformylglycyl)benzoate d'éthyle sous forme d'un solide beige fondant à 105°C.

Le 4-bromoacétylbenzoate d'éthyle peut être préparé selon la méthode décrite dans le brevet EP 44704.

### EXEMPLE H : 4(5)-(2-carbéthoxyphényl)-1H-imidazole-2-carboxylate d'éthyle

A une solution de 2,66 g de thiooxamate d'éthyle dans 100 ml de chlorure de méthylène on ajoute goutte à goutte à 20°C en 10 minutes une solution de 5,7 g de tétrafluoborate de triéthyloxonium dans 25 ml de chlorure de méthylène. Le mélange est agité pendant 16 heures à une température voisine de 20°C puis concentré à sec sous pression réduite (15 mm Hg; 2 kPa) à 40°C. Le résidu (6,4 g) est dissous dans 40 ml d'acide acétique et on ajoute 5,4 g de chlorhydrate de 2-glycylbenzoate d'éthyle puis 3,28 g d'acétate de sodium anhydre. Le mélange est agité pendant 3 heures 30 minutes à 95°C, conservé sans agitation pendant 48 heures à une température voisine de 20°C puis concentré à sec sous pression réduite (15 mm Hg; 2 kPa) à 40°C. Le produit obtenu est additionné de 150 ml de chlorure de méthylène et le mélange est lavé 3 fois avec 150 ml au total d'eau distillée. La solution organique est séchée sur du sulfate de sodium anhydre et concentrée à sec sous pression réduite (15 mm Hg; 2 kpa) à 40°C. Le produit obtenu est chromatographié sur gel de silice neutre en éluant avec de l'acétate d'éthyle. On obtient ainsi 1,6 g de 4(5)-(2-carbéthoxyphényl)-1H-imidazole-2-carboxylate d'éthyle sous forme d'une huile orange [Spectre ¹H dans DMSO-d6, T=300K, δ en ppm (300 MHz): 1,15 (3H, t, J=6Hz, CH₃), 1,35 (3H, t, J=6Hz, CH₃), 4,25 (2H, q, J=6Hz, OCH₂), 4,40 (2H, q, J=6Hz, OCH₂), 7,45 (1H, t, J=7Hz, CH arom), entre 7,50 et 7,85 (4H, m, 3 CH arom. et CH imidazole), 13,60 (1H, s, NH)].

Le chlorhydrate de 2-glycylbenzoate d'éthyle peut être préparé de la manière suivante : 11,2 ml d'une solution aqueuse 10N d'acide chlorhydrique sont ajoutés à 120 mi d'éthanol absolu. A 70 ml de la solution ainsi obtenue, on ajoute 7 g de 2-(N,N-diformylglycyl)benzoate d'éthyle. Le mélange est agité pendant 16 heures à une température voisine de 20°C et concentré à sec sous pression réduite (15 mm Hg; 2 kPa) à 40°C. On obtient ainsi 5,46 g de chlorhydrate de 2-glycylbenzoate d'éthyle sous la forme d'une gomme brune utilisée telle quelle à l'étape suivante.

Le 2-(N,N-diformylglycyl)benzonitrile peut être préparé de la manière suivante : à une solution de 14,45 g de 2-bromoacétylbenzoate d'éthyle dans 150 ml d'acétonitrile on ajoute 5,54 g de diformylamidure de sodium et le mélange est agité pendant 16 heures à l'ébullition puis, après refroidissement à 20°C, l'insoluble apparu est éliminé par filtration et le filtrat est concentré à sec sous pression réduite (15 mm Hg; 2 kPa) à 40°C. Le résidu est chromatographié sur gel de silice neutre en éluant avec un mélange chlorure de méthylène-acétate d'éthyle (80-20 en volumes). On obtient ainsi 8,4 g de 2-(N,N-diformylglycyl)benzoate d'éthyle sous la forme d'une huile orange [Spectre ¹H dans DMSO-d6, T=300K, δ en ppm (300 MHz): 1,22 (3H, t, J=6Hz, CH₃), 4,23 (2H, q, J=6Hz, OCH₂), 4,90 (2H, s, NCH₂CO), entre 7,60 et 7,90 (4H, m, 4 CH arom), 9,20 (2H, s, 2 CHO)].

Le 2-bromoacétylbenzoate d'éthyle peut être préparé selon la méthode décrite par VITI G. et coll., J. Heterocyclic Chem., 28, 379 (1991).

### PREPARATION DES COMPOSES DE FORMULE (I)

### EXEMPLE 1

On chauffe à reflux pendant 6 heures un mélange de 9,54 g de 1-[4-(1(2)-benzyltétrazole-5-yl-méthyl)-1-oxo-indane-2-yl]imidazole-2-carboxylate d'éthyle, 370 ml d'acide acétique et 83 g d'acétate d'ammonium. Le mélange réactionnel est évaporé au rotavapor et le résidu d'évaporation est traité avec 400 ml d'eau distillée. Le précipité gommeux apparu est isolé et trituré avec 150 ml d'acétate d'éthyle. Après filtration et lavage avec 50 ml d'acétate d'éthyle, le solide est séché sous vide (1 mmHg; 0,13 kPa) à une température voisine de 60°C. On obtient 2,5 g de 9-(1 (2)-benzyltétrazole-5-yl-méthyl)-5H,10H-imidazo[1,2-a]indéno[1,2-e]pyrazine-4-one sous forme de solide blanc cassé [RMN Spectre ¹H dans DMSO-d6, T=300K (δ en ppm, 250MHz) : Mélange 75/25 des 2 isomères : Isomère majoritaire : 4,0 (2H, CH₂), 4,4 (2H, s, CH₂), 5,9 (2H, CH₂Ph), entre 7,2 et 7,5 (7H, 2 CH arom. et phényle), 7,6 (1H,s, CH imidazole), 7,8 (1H, d, J=6Hz, CH arom.), 8,0 (1H,s, CH imidazole), 12,4 (1H, s, NH); Isomère minoritaire : 3,95 (2H, CH₂), 4,5 (2H, s, CH₂), 5,8 (2H, CH₂Ph), entre 6,9 et 7,5 (8H, CH arom. et phényle), 7,6 (1H,s, CH imidazole), 7,95 (1H,s, CH imidazole), 12,4 (1H, s, NH)].

On chauffe à une température voisine de 100°C un mélange de 2 g de 9-(1 (2)-benzyltétrazole-5-yl-méthyl)-5H,10H-imidazo[1,2-a]indéno[1,2-elpyrazine-4-one et 100 ml d'acide bromhydrique à 47% dans l'eau pendant 40 heures. Le mélange réactionnel est filtré et le solide est lavé plusieurs fois à l'eau distillée, puis avec un mélange de 10 ml de méthylisobutyléther et 20 ml d'acétone et ensuite avec 2x40 ml d'acétone. Après séchage à l'air le produit brut (1,3 g) est traité aux ultrasons avec 80 ml de soude 0,1 N et la solution brune obtenue est lavée avec 40 ml d'acétate d'éthyle, agitée avec un peu de noir végétal, filtrée et acidifiée à pH 1 avec de l'acide chlorhydrique 1N. La suspension obtenue est filtrée et le solide est lavé avec 2x20 ml d'eau distillée, puis 4x15 ml d'acétone et 3x15 ml de méthylisobutyléther. Après séchage sous vide (1 mmHg; 0,13 kPa) à une température voisine de 60°C on obtient 0,8 g de 9-(1H-tétrazole-5-yl-méthyl)-5H,10H-imidazo[1,2-a]indéno[1,2-e]pyrazine-4-one sous forme de solide gris en partie salifié fondant au-dessus de 260°C [Analyse C₁₅H₁₁N₇O, 0,33 HCI % calculé C : 56,75, H : 3,60, Cl : 3,72, N : 30,89, O : 5,04, % trouvé C : 57,1, H : 3,2, Cl : 4,0, N : 30,8].

Le 1-[4-(1(2)-benzyltétrazole-5-yl-méthyl)-1-oxo-indane-2-yl]imidazole-2-carboxylate d'éthyle peut être préparé de la manière suivante : sous couverture d'argon on porte à reflux un mélange de 0,7 g de 4-(1 (2)-benzyltétrazole-5-yl-méthyl)-2-bromo-indane-1-one, 20 ml d'acétone et 3,33 g de carbonate de potassium. On ajoute alors une solution de 1,92 g d'imidazole-2-carboxylate d'éthyle dans 20 ml d'acétone et l'on poursuit le reflux pendant 2 heures. Le mélange réactionnel est filtré et le filtrat est évaporé au rotavapor. On obtient 2,07 g de 1-[4-(1(2)-benzyltétrazole-5-yl-méthyl)-1-oxo-indane-2-yl]imidazole-2-carboxylate d'éthyle sous forme de meringue noir verdâtre utilisée telle quelle dans les synthèses ultérieures.

L'imidazole-2-carboxylate d'éthyle peut être obtenu comme décrit dans le brevet US 3600399.

La 4-(1(2)-benzyltétrazole-5-yl-méthyl)-2-bromo-indane-1-one peut être préparée de la façon suivante : sous couverture d'argon, à une solution agitée et refroidie à une température voisine de 5°C de 9,8 g de 4-(1(2)-benzyltétrazole-5-yl-méthyl)-indane-1-one dans 100 ml de dichlorométhane on ajoute goutte à goutte une solution de 1,65 ml de brome dans 10 ml de dichlorométhane et on poursuit l'agitation pendant deux heures à une température voisine de 20°. Le mélange réactionnel est lavé avec 4x100 ml de solution saline et la phase organique est séchée sur sulfate de magnésium, filtrée et le filtrat est évaporé au rotavapor. On obtient 12,1 g de 4-(1 (2)-benzyltétrazole-5-yl-méthyl)-2-bromo-indane-1-one sous forme d'huile brune utilisée telle quelle dans les synthèses ultérieures.

La 4-(1 (2)-benzyltétrazole-5-yl-méthyl)-indane-1-one peut être préparée de la manière suivante : sous couverture d'argon on chauffe à reflux pendant 4 heures un mélange de 6,89 g de 4-(1H-tétrazole-5-yl-méthyl)-indane-1-one, 150 ml d'acétone, 4,3 g de carbonate de potassium et 4 ml de bromure de benzyle. Le mélange réactionnel encore chaud est filtré, l'insoluble est rincé avec 3x30 ml d'acétone et le filtrat est évaporé au rotavapor. On obtient 9,8 g de 4-(1(2)-benzyltétrazole-5-yl-méthyl)-indane-1-one sous forme d'huile jaune [Spectre de masse (impact électronique) m/z 350 (M)^{+.}, 213 (350-C₇H₇)⁺, 158 (C₈H₆N₄)^{+.}, 145 (C₁₀H₉O)⁺, 91 (C₇H₇)⁺].

La 4-(1H-tétrazole-5-yl-méthyl)-indane-1-one peut être préparée de la façon suivante : sous couverture d'argon, à 50 ml d'acide sulfurique concentré chauffé à une température voisine de 100°C on ajoute en une fois 9,29 g d'acide 3-[2-(1H-tétrazole-5-yl-méthyl)-phényl]propionique. Le chauffage est poursuivi pendant 50 minutes à une température voisine de 110 °C. Le mélange réactionnel est versé sur 300 g de glace pilée et agité pendant deux heures. La suspension beige obtenue est filtrée et le solide est lavé avec 3x20 ml d'eau distillée et séché sous vide (1 mmHg; 0,13 kPa) à une température voisine de 60°C. On obtient 5,5 g de 4-(1 H-tétrazole-5-yl-méthyl)-indane-1-one sous forme de solide beige clair fondant à 222°C [RMN Spectre ¹H dans DMSO-d6, T=300K (δ en ppm, 300mhz) : 2,65 (2H, m, COCH₂), 3,05 (2H, m, CH₂), 4,40 (2H, s, CH₂), 7,45 (1H, t, J=6Hz, CH arom.), 7,60 (2H,m, 2 CH arom.)].

L'acide 3-[2-(1H-tétrazole-5-yl-méthyl)-phényl]propionique peut être préparé de la manière suivante : une solution de 3,2 g d'acide 3-[2-(1(2)-benzyltétrazole-5-yl-méthyl)-phényl]acrylique dans 50 ml de soude 0,5N est hydrogénée pendant 21 heures à une température voisine de 20°C sous une pression voisine de 1,5 bar, en présence de 0,3 g de charbon palladié à 10%. Le mélange réactionnel est filtré, puis le filtrat est acidifié à pH 1 avec de l'acide chlorhydrique 1N et extrait avec 3x50 ml d'acétate d'éthyle. La phase organique est lavée avec 30 ml d'eau distillée, séchée sur sulfate de magnésium, filtrée et évaporée au rotavapor. On obtient 2,16 g d'acide 3-[2-(1H-tétrazole-5-yl-méthyl)-phényl]propionique sous forme de solide blanc fondant à 160°C.

L'acide 3-[2-(1(2)-benzyltétrazole-5-yl-méthyl)-phényl]acrylique peut être préparé de la façon suivante : sous couverture d'argon on chauffe pendant 16 heures à une température voisine de 100°C un mélange de 117,5 g de 1(2)-benzyl-5-(2-bromobenzyl)tétrazole, 162 ml de tributylamine, 4,14 g de tri(o-tolyl)phosphine, 0,76 g d'acétate de palladium et 29,2 d'acide acrylique. Le milieu réactionnel est versé sur un mélange agité de 700 ml d'acide chlorhydrique 1N et de 1400 ml d'acétate d'éthyle. La phase organique est lavée avec 2x400 ml d'eau distillée, puis avec 2x750 ml d'eau contenant 20 g de carbonate de sodium. Les phases aqueuse sont réunies, acidifiées à pH 1 avec de l'acide chlorhydrique 6N et extraites avec 2x500 ml d'acétate d'éthyle. L'extrait organique est lavé avec 250 ml d'eau distillée, séché sur sulfate de magnésium, filtré et évaporé au rotavapor. On obtient 77,8 g d'acide 3-[2-(1 (2)-benzyltétrazole-5-yl-méthyl)-phényl]acrylique sous forme de solide crème utilisé tel quel dans les synthèses ultérieures.

Le 1(2)-benzyl-5-(2-bromobenzyl)tétrazole peut être préparé de la manière suivante : sous couverture d'argon on chauffe à reflux pendant 5 heures un mélange de 81,5 g de 5-(2-bromobenzyl)tétrazole, 800 ml d'acétone, 45,4 g de carbonate de potassium et 44,6 ml de bromure de benzyle. Le mélange réactionnel est filtré et le solide est rincé avec 2x100 ml d'acétone, puis le filtrat est évaporé au rotavapor. On obtient 117,5 g de 1(2)-benzyl-5-(2-bromobenzyl)tétrazole sous forme de liquide orange utilisé tel quel dans les synthèses ultérieures.

Le 5-(2-bromobenzyl)tétrazole peut être préparé de la façon suivante : sous couverture d'argon on chauffe pendant 3 heures à une température voisine de 100°C, puis pendant 3 heures à reflux un mélange de 80 g de (2-bromophényl)acétonitrile, 160 ml de diméthylformamide, 29,2 g d'azoture de sodium et 24 g de chlorure d'ammonium. Après arrêt du chauffage on ajoute 29,2 g d'azoture de sodium et 24 g de chlorure d'ammonium et on chauffe à nouveau pendant 16 heures à une température voisine de 100°C. Le mélange réactionnel est versé sur 2 litres d'un mélange d'eau et de glace, acidifié à pH 4-5 avec 60 ml d'acide acétique et agité pendant 2 heures à une température voisine de 20°C. La suspension crème obtenue est filtrée et le solide est lavé avec 150 ml d'eau distillée, agité avec 2 litres d'eau contenant 45 g de carbonate de sodium et extrait avec 2x250 ml de méthyltert-butyléther. La phase aqueuse est filtrée et acidifiée à pH 1 avec de l'acide chlorhydrique 6N. La suspension blanche obtenue est filtrée et le solide est lavé avec 200 ml d'eau distillée et séché sous vide (1 mmHg; 0,13 kPa) au voisinage de 60°C. On obtient 81,5 g (2-bromobenzyl)tétrazole sous forme de solide blanc fondant à 139°C.

### EXEMPLE 2

On chauffe à reflux pendant 4 heures un mélange de 11,2 g de 1-[4-(1(2)-benzyltétrazole-5-yl-méthyl)-1-oxo-indane-2-yl]-imidazole-2,4-dicarboxylate de diéthyle, 400 ml d'acide acétique et 87,5 g d'acétate d'ammonium. Le mélange réactionnel est concentré au rotavapor et le résidu est agité pendant 15 minutes avec 750 ml d'eau distillée. La suspension brune obtenue est filtrée et le solide est lavé successivement avec 2x125 ml d'eau distillée et 4x100 ml d'acétate d'éthyle. Après séchage à l'air on obtient 3,87 g de 9-(1 (2)-benzyltétrazole-5-yl-méthyl)-4,5-dihydro-4-oxo-10H-imidazo[1,2-a]indéno[1,2-e]pyrazine-2-carboxylate d'éthyle sous forme de solide brun [RMN Spectre ¹H dans DMSO-d6, T=300K (δ en ppm, 250mhz) : Mélange 60/40 des 2 isomères : Isomère majoritaire : 1,4 (3H, CH₃), 4,0 (2H, CH₂), entre 4,3 et 4,6 (4H, CH₂ et OCH₂), 5,9 (2H, CH₂Ph), entre 7,2 et 7,9 (5H, CH phényle), 8,5 (1H,s, CH imidazole), 12,5 (1H, s, NH); Isomère minoritaire: 1,4 (3H, CH₃), 3,9 (2H, CH₂), entre 4,3 et 4,6 (4H, CH₂ et OCH₂), 5,8 (2H, CH₂Ph), entre 7,0 et 7,6 (5H, CH phényle), 8,5 (1H,s, CH imidazole), 12,5 (1H, s, NH)].

On chauffe pendant 20 heures à une température voisine de 100°C un mélange de 3 g de 9-(1(2)-benzyltétrazole-5-yl-méthyl)-4,5-dihydro-4-oxo-10H-imidazo[1,2-a]indéno[1,2-e]pyrazine-2-carboxylate d'éthyle et 150 ml d'acide bromhydrique à 47% dans l'eau. Le mélange réactionnel est filtré et le solide est lavé avec 4x50 ml d'eau distillée, puis 4x50 ml d'acétone et enfin 2x50 ml de méthyltert-butyléther. Le solide est alors agité en présence d'ultrasons avec 65 ml de soude 0,1N et la phase aqueuse est lavée avec 30 ml d'acétate d'éthyle, additionnée de 0,13 g de charbon végétal et filtrée. Le filtrat est acidifié à pH 1 avec de l'acide chlorhydrique 1N et le précipité formé est filtré et séché sous vide (1 mmHg; 0,13 kPa) au voisinage de 60°C. On obtient 0,94 g d'acide 9-(1H-tétrazole-5-yl-méthyl)-4,5-dihydro-4-oxo-10H-imidazo[1,2-a]indéno[1,2-e]pyrazine-2-carboxylique sous forme de solide rose pâle fondant au-dessus de 260°C [Analyse C₁₆H₁₁N₇O₃ % calculé C : 55,02, H : 3,17, N : 28,07, O : 13,74, % trouvé C : 55,3, H : 2,8, N : 27,9].

Le 1-[4-(1 (2)-benzyltétrazole-5-yl-méthyl)-1-oxo-indane-2-yl]-imidazole-2,4-dicarboxylate de diéthyle peut être préparé de la manière suivante : sous couverture d'argon on porte à reflux un mélange de 5,3 g d'imidazole-2,4-dicarboxylate de diéthyle, 100 ml d'acétone et 16,7 g de carbonate de potassium. On ajoute alors une solution de 9,58 g de 4-(1(2)-benzyltétrazole-5-yl)méthyl-2-bromo-indane-1-one dans 50 ml d'acétone et on poursuit le reflux pendant 4 heures. Le mélange réactionnel est filtré, l'insoluble est lavé avec 3x100 ml d'acétone et la phase organique est évaporée au rotavapor. On obtient 11,2 g de 1-[4-(1(2)-benzyltétrazole-5-yl-méthyl)-1-oxo-indane-2-yl]-imidazole-2,4-dicarboxylate de diéthyle sous forme de meringue noire utilisée telle quelle dans les synthèses ultérieures.

L'imidazole-2,4-dicarboxylate de diéthyle peut être synthétisé comme décrit par P.S. BRANCO et coll., Tetrahedron, 48(30), 6335 (1992).

### EXEMPLE 3

On chauffe à reflux pendant 38 heures un mélange de 3,8 g de 1-[4-(1(2)-benzyltétrazole-5-yl-méthyl)-1-oxo-indane-2-yl-]-4-(diéthoxy-phosphoryl)-imidazole-2-carboxylate d'éthyle, 33 ml d'acide acétique, 21 ml de solution ammoniacale 5N dans le méthanol et 5 g d'acétate d'ammonium. Le mélange réactionnel est concentré au rotavapor et le résidu est additionné de 100 ml d'eau distillée et extrait avec 2x50 ml d'acétate d'éthyle. La phase organique est séchée sur sulfate de magnésium, filtrée et évaporée au rotavapor. Le résidu d'évaporation est agité pendant la nuit avec 80 ml de méthyltert-butyléther et 8 ml d'acétate d'éthyle à une température voisine de 20°C et filtré. Après séchage à l'air on obtient 1,5 g de 9-(1 (2)-benzyltétrazole-5-yl-méthyl)-4,5-dihydro-4-oxo-1 OH-imidazo[1,2-a]indéno[1,2-e]pyrazine-2-phosphonate de diéthyle sous forme de solide beige clair [RMN Spectre ¹H dans DMSO-d6, T=300K (δ en ppm, 300mhz) : Mélange 70/30 des 2 isomères : Isomère majoritaire : 1,3 (6H, t, J=6Hz, 2 CH₃), 4,0 (2H, s, CH₂), 4,15 (4H, m, OCH₂), 4,4 (2H, s, CH₂), 5,9 (2H, s, CH₂), entre 7,2 et 7,6 (7H, m, CH arom. et phényle), 7,8 (1H, d, J=6Hz, CH arom.), 8,45 (1H,s, CH imidazole), 12,5 (1H, s, NH); Isomère minoritaire : 1,3 (6H, t, J=6Hz, 2 CH₃), 3,95 (2H, s, CH₂), 4,15 (4H, m, OCH₂), 4,5 (2H, s, CH₂), 5,8 (2H, s, CH₂), entre 7,0 et 7,6 (8H, m, CH arom. et phényle), 8,45 (1H,s, CH imidazole), 12,5 (1H, s, NH)].

Sous couverture d'argon on chauffe à une température voisine de 110°C pendant 2 heures, puis à 100°C pendant 16 heures un mélange de 1,15 g de 9-(1 (2)-benzyltétrazole-5-yl-méthyl)-4,5-dihydro-4-oxo-1 OH-imidazo[1,2-a]indéno[1,2-e]pyrazine-2-phosphonate de diéthyle et 60 mi d'acide bromhydrique à 47% dans l'eau. Le mélange réactionnel est filtré et le solide est lavé avec 4x10 ml d'eau distillée, puis 3x40 ml de méthyltert-butyléther. Après séchage sous vide (1 mmHg; 0,13 kPa) au voisinage de 60°C on obtient 0,43 g d'acide 9-(1H-tétrazole-5-yl-méthyl)-4,5-dihydro-4-oxo-10H-imidazo[1,2-a]indéno[1,2-e]pyrazine-2-phosphonique sous forme de solide rose fondant au-dessus de 260°C [Analyse C₁₅H₁₂N₇O₄P % calculé C : 46,76, H : 3,14, N : 25,45, O : 16,61, P : 8,04, % trouvé C : 46,7, H : 2,8, N : 25,1].

Le 1-[4-(1(2)-benzyltétrazole-5-yl-méthyl)-1-oxo-indane-2-yl-]-4-(diéthoxyphosphoryl)-imidazole-2-carboxylate d'éthyle peut être préparé de la manière suivante : on procède comme à l'exemple 2 pour la préparation du 1-[4-(1(2)-benzyltétrazole-5-yl-méthyl)-1-oxo-indane-2-yl]-imidazole-2,4-dicarboxylate de diéthyle, mais à partir de 2,76 g de 4-(diéthoxyphosphoryl)-imidazole-2-carboxylate d'éthyle, 100 ml d'acétone, 6,7 g de carbonate de potassium et 3,83 g de 4-(1(2)-benzyltétrazole-5-yl-méthyl)-2-bromo-indane-1-one. Le produit brut (5,7 g) est purifié par chromatographie sur colonne de silice en éluant avec un mélange de dichlorométhane et de méthanol (95-5 en volumes). On obtient 3,8 g de 1-[4-(1(2)-benzyltétrazole-5-yl-méthyl)-1-oxo-indane-2-yl-]-4-(diéthoxyphosphoryl)-imidazole-2-carboxylate d'éthyle sous forme de meringue brune utilisée telle quelle dans les synthèses ultérieures.

### EXEMPLE 4

On chauffe à reflux pendant 5 heures un mélange de 1,63 g de 1-[4-(éthoxycarbonylméthyl)-1-oxo-indane-2-yl]-4-(éthoxycarbonylméthyl)-imidazole-2-carboxylate d'éthyle, 15 ml d'acide acétique et 2,83 g d'acétate d'ammonium. On arrête le chauffage, on ajoute 1,4 g d'acétate d'ammonium et on poursuit le reflux pendant 2 heures. Le mélange réactionnel est versé sur 100 ml de glace pilée et extrait avec 3x50 ml de dichlorométhane. La phase organique est séchée sur sulfate de magnésium, filtrée et évaporée au rotavapor. Le résidu d'évaporation est mis en suspension dans 15 ml d'acétate d'éthyle pour donner 0,8 g de 4,5-dihydro-4-oxo-10H-imidazo[1,2-a]indéno[1,2-e]pyrazine-2,9-diacétate de diéthyle sous forme de solide beige [RMN Spectre ¹H dans DMSO-d6, T=300K, δ en ppm (250 Mhz) : 1,25 (6H, m, 2 CH₃), 3,80 (4H, s, 2 CH₂CO₂Et), 4,00 (2H, s, CH₂), 4,15 (4H, q, J=6Hz, 2 OCH₂), 7,15 (1H, d, J=8Hz, CH arom), 7,40 (1H, t, J=8Hz, CH arom.), 7,80 (1H, d, J=8Hz, CH arom.), 7,90 (1H, s, CH arom.), 12,4 (1H, s, CONH)].

On chauffe pendant 16 heures à une température voisine de 100°C un mélange de 0,68 g de 4,5-dihydro-4-oxo-10H-imidazo[1,2-a]indéno[1,2-e]pyrazine-2,9-diacétate de diéthyle et 20 ml d'acide chlorhydrique 6N. Le mélange réactionnel est refroidi dans un bain d'eau et de glace et la suspension est filtrée. L'insoluble est lavé avec 3x10 ml d'acétone et séché sous vide (1 mmHg; 0,13 kPa) au voisinage de 60°C. On obtient 0,43 g du chlorhydrate de l'acide 4,5-dihydro-4-oxo-10H-imidazo[1,2-a]indéno[1,2-e]pyrazine-2,9-diacétique sous forme de solide beige foncé fondant au-dessus de 250°C [Analyse C₁₇H₁₃N₃O₅, HCl % calculé C : 54,34, H : 3,76, Cl : 9,43, N : 11,18, O : 21,29, % trouvé C : 53,9, H : 3,4, Cl : 9,8, N : 10,9, O : 20,9].

Le 1-[4-(éthoxycarbonylméthyl)-1-oxo-indane-2-yl]-4-(éthoxycarbonylméthyl)-imidazole-2-carboxylate d'éthyle peut être préparé de la manière suivante : on porte à reflux un mélange de 1,37 g de 4-(éthoxycarbonylméthyl)-imidazole-2-carboxylate d'éthyle, 30 ml d'acétone et 4,14 g de carbonate de potassium. On ajoute alors en 10 minutes une solution de 1,78 g de (2-bromo-1-oxo-indane-4-yl)acétate d'éthyle dans 10 ml d'acétone et on continue le reflux pendant 3 heures. Le mélange réactionnel encore chaud est filtré et le filtrat est évaporé au rotavapor. Le résidu d'évaporation est purifié par chromatographie sur colonne de silice en éluant avec de l'accétate d'éthyle. On obtient 1,65 g de 1-[4-(éthoxycarbonylméthyl)-1-oxo-indane-2-yl]-4-(éthoxycarbonylméthyl)-imidazole-2-carboxylate d'éthyle sous forme d'huile brune [RMN Spectre ¹H dans DMSO-d6, T=300K, δ en ppm (300 Mhz) : 1,20 (9H, m, 3 CH₃), 3,25 (1H, dd, J=5 et 14Hz, HCH), 3,70 (2H, s, CH₂CO), 3,75 (1H, dd, J=7 et 14Hz, HCH), 3,85 (2H, s, CH₂CO), 4,10 (6H, m, 3 OCH₂), 5,80 (1H, dd, J=5 et 7Hz, CH), 7,50 (2H, m, 2 CH arom), 7,70 (2H, d, J=8Hz, 2 CH arom.)].

Le (2-bromo-1-oxo-indane-4-yl)acétate d'éthyle peut être préparé de la manière suivante : à 10,45 g d'ester éthylique de l'acide (1-oxo-indane-4-yl)acétique dans 130 ml de dichlorométhane sont ajoutés, en 10 minutes, 2,15 ml d'une solution de brome dans 20 ml de dichlorométhane à une température voisine de 5°C et sous atmosphère d'argon. On laisse revenir le milieu réactionnel à une température voisine de 20°C et poursuit la réaction pendant 2 heures. Le mélange réactionnel est versé dans 100 ml d'eau saturée en chlorure de sodium. La phase organique est lavée par deux fois 100 ml d'eau distillée avant d'être séchée et concentrée pour conduire à 12,7 g de produit attendu sous forme d'une huile brune utilisée telle quelle dans les synthèses ultérieures.

L'ester éthylique de l'acide (1-oxo-indane-4-yl)acétique peut être préparé de la manière suivante : à 9,4 g d'acide (1-oxo-indane-4-yl)acétique dans 200 ml de dichlorométhane sont ajoutés 4,7 ml de chlorure d'oxalyle à température ambiante et sous atmosphère d'argon. Après 4 heures d'agitation à une température voisine de 20°C, 40 ml d'éthanol sont ajoutés au milieu réactionnel et on maintient l'agitation pendant 1 heure. La phase organique est lavée par 2 fois 25 ml d'une solution saturée en hydrogénocarbonate de sodium puis par 2 fois 100 ml d'eau distillée puis séchée et concentrée pour conduire à 10,45 g de produit attendu sous forme d'une huile brune utilisée telle quelle dans les synthèses ultérieures.

L'acide (1-oxo-indane-4-yl)acétique peut être préparé de la manière suivante : 43 g d'acide 3-(2-carboxyméthyl-phényl)propionique dans 250 ml d'acide sulfurique (95%) sont chauffés à 100°C pendant 18 heures. Après refroidissement à une température voisine de 20°C le milieu réactionnel est versé sur 1000 ml d'eau glacée. Le milieu est extrait par trois fois 400 ml d'acétate d'éthyle et la phase organique est lavée à l'eau, séchée, concentrée pour conduire à 9,56 g d'un solide orangé. Le produit ainsi obtenu est mis en suspension dans 50 ml d'éther de pétrole, filtré puis lavé par 25 ml d'éther isopropylique, séché sous pression réduite (1mm Hg; 0,13 kPa) à 60°C pour conduire à 6,7 g d'un solide jaune orangé fondant à 160°C.

L'acide 3-(2-carboxyméthyl-phényl)propionique peut être préparé de la manière suivante : 39,6 g d'acide 3-(2-carboxyméthyl-phényl)acrylique avec 3 g de charbon palladié à 10% dans 400 ml d'acide acétique sont hydrogénés à une température voisine de 20°C sous une pression de 1,2 bar pendant 4 heures. Après filtration du milieu réactionnel, la phase organique est concentrée sous pression réduite (15 mm Hg; 2 kPa) à 40°C. Le solide blanc obtenu est mis en suspension dans 100 ml d'éther de pétrole puis filtré et séché sous pression réduite (1mm Hg; 0,13 kPa) à 20°C pour conduire à 39,3 g d'un solide blanc fondant à 138°C.

L'acide 3-(2-carboxyméthyl-phényl)acrylique peut être préparé de la manière suivante : 63,8 g d'acide 2-bromophényl acétique, 25,5 ml d'acide acrylique, 3,6 g de tri(2-tolyl)phosphine, 0,67 g d'acétate de palladium dans 211 ml de tributylamine sont chauffés à 100°C pendant 6 heures. Après refroidissement à une température voisine de 20°C le milieu réactionnel est versé sur 420ml d'eau et 80 ml d'acide chlorhydrique concentré. Le milieu est extrait par 3 fois 500 ml d'acétate d'éthyle; la phase organique est filtrée, lavée par 3 fois 500 ml d'eau puis agitée en présence de 800 ml d'eau et 35 g de carbonate de sodium à une température voisine de 20°C pendant 15 minutes. La phase aqueuse est ensuite acidifiée par 700 ml d'acide chlorhydrique 1N. Le précipité formé est filtré, lavé à l'eau puis séché sous pression réduite (1mm Hg; 0,13 kPa) à 60°C pour conduire à 39,6 g d'un solide blanc fondant à 190°C.

### EXEMPLE 5

Un mélange de 3,13 g de 1-(4-cyanométhyl-1-oxo-indane-2-yl)imidazole-2-carboxylate d'éthyle, de 30 ml d'acide acétique et de 20 ml de méthanol ammoniacal 5N est porté au reflux pendant 20 heures. Après refroidissement à une température voisine de 20°C, le milieu réactionnel est concentré et le résidu ainsi obtenu est repris par 150 ml d'eau. Le précipité ainsi formé est filtré sur verre fritté, rincé à l'eau, puis séché sous pression réduite à 20°C. On obtient ainsi 2,6 g d'un solide marron. Ce solide est repris sous agitation par 30 ml d'un mélange dichlorométhane-méthanol (95/5 en volumes), filtré sur verre fritté, rincé par 2x30 ml du même mélange puis séché sous pression réduite à 60°C. On obtient ainsi 0,84 g de 9-cyanométhyl-5H,10H-imidazo[1,2-a]indéno[1,2-e]pyrazine-4-one sous forme de solide beige fondant au-dessus de 260°C [Analyse C₁₅H₁₀N₄O, 0,2 H₂O, 0,17 CH₃COOH % calculé C : 68,70, H : 3,84,N : 21,36, O : 6,10, % trouvé C : 69,1, H : 3,5, N : 20,8].

Le 1-(4-cyanométhyl-1-oxo-indane-2-yl)imidazole-2-carboxylate d'éthyle peut être obtenu de la manière suivante : une solution de 1,8 g d'imidazole-2-carboxylate d'éthyle dans 30 ml d'acétone est additionnée de 8,6 g de carbonate de potassium. Cette suspension est portée au reflux pendant 15 minutes, puis est ajoutée une solution de 3,22 g de 2-bromo-4-(cyanométhyl)indane-1-one dans 30 ml d'acétone. Après 4 heures d'agitation au reflux, le milieu réactionnel est ramené à une température voisine de 20°C et filtré sur verre fritté. Le filtrat est évaporé et on obtient 2,7 g d'un solide noir. La purification par chromatographie flash sur colonne de silice (éluant: dichlorométhane-acétate d'éthyle (50-50 en volumes)) de ce solide conduit à 0,62 g de 1-(4-cyanométhyl-1-oxo-indane-2-yl)imidazole-2-carboxylate d'éthyle sous forme de solide brun [spectre de masse m/z 309 (M^{+.}), 236 ((M-CO₂Et)⁺), 141 ((C₆H₉N₂)⁺), 68 ((C₃H₄)⁺)].

L'imidazole-2-carboxylate d'éthyle peut être préparé comme décrit dans le brevet US 3600399.

La 2-bromo-4-(cyanométhyl)indane-1-one peut être synthétisée de la façon suivante : à une solution de 2,42 g de 4-(cyanométhyl)indane-1-one dans 25 ml de dichlorométhane est ajoutée à 5°C une solution de 0,72 ml de brome dans 5 ml de dichlorométhane goutte à goutte. Après 3 heures à une température voisine de 22°C, le milieu réactionnel est repris par 30 ml d'eau salée, agité puis décanté. La phase organique est lavée par 3x30 ml d'eau salée, séchée sur sulfate de magnésium et évaporée. On obtient ainsi 3,22 g de 2-bromo-4-(cyanométhyl)indane-1-one sous forme de solide beige [Rf = 0,36; chromatographie couche mince sur gel de silice, éluant : cyclohexane-acétate d'éthyle (2/1 en volumes)].

La 4-(cyanométhyl)indane-1-one peut être préparée de la façon suivante : à une solution de 3 ml de chlorure de thionyle et 15 ml de dichlorométhane sont additionnés 3,78 g d'acide 3-(2-cyanométhyl-phényl)propanoïque. Après avoir ajouté 0,55 ml de diméthylformamide, le milieu réactionnel est porté à 30°C pendant 4 heures. L'évaporation du milieu conduit à une huile orange. Cette huile est solubilisée dans 25 ml de 1,2-dichloroéthane, puis est additionnée goutte à goutte à 10°C sous atmosphère d'argon à une solution de 8 g de chlorure d'aluminium et de 35 ml de 1,2-dichloroéthane. Après 18 heures de réaction à une température voisine de 20°C, le milieu réactionnel est versé sur 60 g de glace. La solution est ensuite décantée et la phase aqueuse est extraite par 50 ml de dichlorométhane. Les phases organiques réunies sont lavées par 100 ml d'une solution aqueuse saturée en bicarbonate de sodium, par 50 ml d'eau puis séchées sur sulfate de magnésium et évaporées. Le résidu solide ainsi obtenu est trituré dans 50 ml de méthyltertbutyléther et la suspension obtenue est filtrée sur verre fritté, rincée par 25 ml de méthyltertbutyléther et sechée sous pression réduite à 20°C. On obtient ainsi 2,42 g de 4-(cyanométhyl)indane-1-one sous forme de solide beige [Spectre R.M.N. ¹H dans DMSO-d6, T=300K, δ en ppm (300 MHz) : 2,70 (2H, m, CH₂), 3,15 (2H, m, CH₂), 4,20 (2H, s, CH₂CN), 7,52 (1H, t, J=8Hz, CH arom.), 7,67 (1H, d, J=8Hz, CH arom), 7,73 (2H, d, J=8Hz, CH arom.)].

L'acide 3-(2-cyanométhyl-phényl)propanoïque peut être préparé de la manière suivante : à une solution de 15,4 g d'acide 3-(2-cyanométhyl)cinnamique dans 250 ml d'eau et 83 ml de soude 1N, sont additionnés 1,5 g de palladium sur charbon à 10%. Cette solution est placée sous hydrogène à une pression voisine de 1,5 bar et à une température voisine de 20°C pendant 2 heures et 45 minutes. Le milieu réactionnel est ensuite filtré sur papier. Le filtrat est acidifié à pH voisin de 1 par ajout de 80 ml d'acide chlorhydrique aqueux (1N). Le précipité ainsi obtenu est filtré sur verre fritté, lavé par 2 x 75 ml d'eau et séché sous pression réduite à une température voisine de 60°C. On obtient ainsi 13,46 g d'acide 3-(2-cyanométhyl-phényl )propanoïque sous forme de solide blanc utilisé tel quel dans les synthèses ultérieures.

L'acide 3-(2-cyanométhyl)cinnamique peut être obtenu de la manière suivante : dans un ballon sous atmosphère d'azote sont introduits successivement 13 ml de 2-bromophényl acétonitrile, 47,5 ml de tributylamine, 1,2 g de tri-o-tolylphosphine, 0,22 g d'acétate de palladium, puis 8,6 ml d'acide acrylique. Le mélange est chauffé 18 heures à 100°C. 5 Après retour à une température voisine de 20°C, le milieu réactionnel est repris par 150 ml d'acétate d'éthyle et 220 ml d'acide chlorhydrique aqueux 1N. Après agitation, le milieu réactionnel est décanté et la phase aqueuse est extraite par 150 ml d'acétate d'éthyle. Les phases organiques sont réunies, lavées par 2x200 ml d'eau, filtrées et reprises par une solution de 10,6 g de 0 bicarbonate de sodium dans 300 ml d'eau. Après une heure d'agitation et décantation, la phase aqueuse est acidifiée par 17,5 ml d'acide chlorhydrique aqueux (12N). Le précipité ainsi obtenu est filtré sur verre fritté, lavé par 2x100 ml d'eau et séché sous pression réduite à une température voisine de 20°C. On obtient ainsi 15,4 g d'acide 3-(2-cyanométhyl)cinnamique sous forme de solide blanc utilisé tel quel dans les synthèses ultérieures.

### EXEMPLE 6

Une solution de 2,15 g de 1-(4-éthoxycarbonylméthyl-1-oxo-indane-2-yl)-2-éthoxycarbonyl-imidazole-4-méthylphosphonate de diéthyle et de 3,23 g d'acétate d'ammonium dans 20 ml d'acide acétique est agitée à l'ébullition pendant 2 heures puis pendant 7 heures après une nouvelle addition de 1,4 g d'acétate d'ammonium. Le mélange est refroidi à une température voisine de 20°C et le solide est séparé par filtration, lavé 2 fois avec 20 ml au total d'acide acétique, 3 fois avec 60 ml au total d'éther éthylique anhydre et séché sous pression réduite. On obtient ainsi 0,72 g de 9-éthoxycarbonylméthyl-4-oxo-4,5-dihydro-10H-imidazo[1,2-a]indéno[1,2-e]pyrazine-2-méthylphosphonate de diéthyle sous forme d'une poudre grise [RMN Spectre ¹H dans DMSO-d6, T=300K, δ en ppm (300 Mhz) : 1,25 (9H, m, 3 CH₃), 3,40 (2H, d, J=19Hz, PCH₂), 3,80 (2H, s, CH₂), 4,00 (2H, s, CH₂), 4,10 (4H, m, 2 OCH₂), 7,20 (1H, d, J=8Hz, CH arom.), 7,40 (1H, t, J=8Hz, CH arom), 7,80 (2H, m, CH arom. et imidazole)].

Une solution de 0,7 g de 9-éthoxycarbonylméthyl-4-oxo-4,5-dihydro-10H-imidazo[1,2-a]indéno[1,2-e]pyrazine-2-méthylphosphonate de diéthyle dans 18 ml d'une solution aqueuse 6N d'acide chlorhydrique est agitée à l'ébullition pendant 16 heures puis refroidie à une température voisine de 20°C. Le solide est séparé par filtration, lavé 3 fois avec 60 ml au total d'eau distillée, 4 fois avec 80 ml au total d'acétone, 4 fois avec 120 ml au total d'éther éthylique anhydre et séché sous pression réduite à 60°C. On obtient ainsi 0,4 g d'acide 9-carboxyméthyl-4-oxo-4,5-dihydro-10H-imidazo[1,2-a]indéno[1,2-e]pyrazine-2-méthylphosphonique sous forme d'une poudre beige fondant au-dessus de 260°C [RMN Spectre ¹H dans D₂O+NaOD, T=300K, δ en ppm (250 Mhz) : 3,05 (2H, d, J=19Hz, PCH₂), 3,30 (2H, s, CH₂), 3,40 (2H, s, CH₂), 6,90 (1H, d, J=8Hz, CH arom), 7,22 (1H, t, J=8Hz, CH arom.), 7,38 (1H, d, J=2Hz, CH imidazole), 7,50 (1H, d, J=8Hz, CH arom.)]

Le 1-(4-éthoxycarbonylméthyl-1-oxo-indane-2-yl)-2-éthoxycarbonyl-imidazole-4-méthylphosphonate de diéthyle peut être préparé de la manière suivante : on ajoute 4,5 g de carbonate de potassium à une solution de 1,95 g de 2-éthoxycarbonylimidazole-4-méthylphosphonate de diéthyle dans 35 ml d'acétone. La suspension agitée est maintenue à l'ébullition puis on ajoute goutte à goutte en 10 minutes une solution de 1,9 g de (2-bromo-1-oxo-indane-4-yl)-acétate d'éthyle dans 10 ml d'acétone. Après 2 heures d'agitation, la solution bouillante est filtrée et le filtrat est concentré à sec sous pression réduite à 40°C. Le produit obtenu (3,2 g) est chromatographié sur gel de silice neutre en éluant avec de l'acétate d'éthyle puis avec un mélange d'acétate d'éthyle et de méthanol (95-5 en volumes). Les fractions contenant le produit attendu sont réunies et concentrées à sec sous pression réduite et on obtient ainsi 2,15 g de 1-(4-éthoxycarbonylméthyl-1-oxo-indane-2-yl)-2-éthoxycarbonyl-imidazole-4-méthylphosphonate de diéthyle fondant à 129°C [RMN Spectre ¹H dans DMSO-d6, T=300K, δ en ppm (250 MHz): entre 1,10 et 1,40 (12H, m, 4 CH₃), 3,25 (2H, d, J=19Hz, PCH₂), 3,27 et 3,78 (1H chacun, m, CH₂), 3,90 (2H, s, CH₂CO), entre 4,00 et 4,30 (8H, m, 4 OCH₂), 5,90 (1H, dd, J=4 et 5Hz, NCH), 7,50 (1H, d, J=2Hz, CH imidazole.), 7,55 (1H, t, J=8Hz, CH arom), 7,82 (2H, d, J=8Hz, 2 CH arom.)].

### EXEMPLE 7

A une suspension de 1,03 g de 1-[1-oxo-4-(2-oxo-2-phényléthoxycarbonylméthyl)-indan-2-yl]-imidazole-2,4-dicarboxylate de diéthyle dans 7,2 ml d'acide acétique, on introduit, sous atmosphère d'argon, 1,54 g d'acétate d'ammonium. Le milieu réactionnel est porté au reflux pendant 4 heures et filtré à chaud sur verre fritté. Le résidu solide obtenu est lavé par deux fois à l'eau puis séché sous pression réduite à 60°C. On obtient ainsi 0,4 g de 9-(4-phényl-1H-imidazol-2-yl-méthyl)-4,5-dihydro-4-oxo-10H-imidazo[1,2-a]indéno[1,2-e]pyrazine-2-carboxylate d'éthyle sous forme de solide gris fondant à une température supérieure à 260°C.

On chauffe pendant 24 heures à une température voisine de 100°C un mélange de 0,4 g de 9-(4-phényl-1H-imidazol-2-yl-méthyl)-4,5-dihydro-4-oxo-10H-imidazo[1,2-a]indéno[1,2-e]pyrazine-2-carboxylate d'éthyle, 12,5 ml d'acide acétique et 2,5 ml d'acide chlorhydrique 6N. Le milieu réactionnel est filtré à chaud sur verre fritté et le résidu solide obtenu est lavé par trois fois à l'eau puis séché sous pression réduite à 60°C. On obtient ainsi 0,23 g d'acide 9-(4-phényl-1 H-imidazol-2-yl-méthyl)-4,5-dihydro-4-oxo-10H-imidazo[1,2-a]indéno [1,2-e]pyrazine-2-carboxylique sous forme de solide blanc fondant à une température supérieure à 260°C (Analyse C₂₄H₁₇N₅O₃, 3,96 H₂O, 0,91 C₂H₄O₂ : % calculé C : 68,07, H : 4,05, N : 16,54, % trouvé C : 68,1, H : 4,1, N : 16,5).

Le 1-[1-oxo-4-(2-oxo-2-phényl-éthoxycarbonylméthyl)-indan-2-yl]-imidazole-2,4-dicarboxylate de diéthyle peut être obtenu de la manière suivante : à une suspension de 4,3 g d'imidazole-2,4-dicarboxylate de diéthyle, de 1,1 g de carbonate de potassium et de 25 ml d'acétone, on ajoute goutte à goutte sous atmosphère d'argon, une solution de 1,8 g d'ester (2-oxo-2-phényl)éthylique de l'acide (2-bromo-1-oxo-indane-4-yl)acétique dans 45 ml d'acétone. Après 24 heures d'agitation à une température voisine de 20°C, le milieu réactionnel est filtré sur verre fritté et le solide résultant est lavé trois fois à l'eau, puis séché sous pression réduite à 60°C. On obtient ainsi 2,63 g de 1-[1-oxo-4-(2-oxo-2-phényl-éthoxycarbonylméthyl)-indan-2-yl]-imidazole-2,4-dicarboxylate de diéthyle sous forme de solide crème fondant à 180°C.

L'ester (2-oxo-2-phényl)éthylique de l'acide (2-bromo-1-oxo-indane-4-yl)acétique peut être obtenu de la manière suivante : à 12,5 g d'ester (2-oxo-2-phényl)éthylique de l'acide (1-oxo-indane-4-yl)acétique dans 200 ml d'acide acétique on ajoute 0,8 ml d'acide bromhydrique. A cette solution refroidie à une température voisine de 13°C, sont ajoutés goutte à goutte et en 5 minutes, 2,35 ml de brome. On laisse revenir le milieu réactionnel à une température voisine de 20°C et poursuit la réaction pendant 2 heures. Cette opération est répétée deux fois. Les deux milieux réactionnels sont réunis, versés dans un mélange de 400 g de glace et 400 ml d'eau et repris par un litre d'éther diéthylique. La phase organique est concentrée et l'huile brune résultante est reprise par 500 ml d'éther diéthylique. Après décantation, la phase éthérée est concentrée et purifiée par chromatographie sur silice avec un mélange cyclohexane-méthanol (7-3 en volumes) comme éluant. On obtient ainsi 9,5 g d'ester (2-oxo-2-phényl)éthylique de l'acide (2-bromo-1-oxo-indane-4-yl)acétique sous forme d'huile jaune utilisée telle quelle dans les synthèses ultérieures.

L'ester (2-oxo-2-phényl)éthylique de l'acide (1-oxo-indane-4-yl)acétique peut être obtenu de la manière suivante : à une solution de 18 g d'acide (1-oxo-indane-4-yl)acétique, de 13,2 ml de triéthylamine dans 190 ml d'acétate d'éthyle, est ajoutée goutte à goutte et à une température maintenue vers 20°C, une solution de 18,92 g de bromoacétophénone dans 47 ml d'acétate d'éthyle. Le milieu réactionnel est agité pendant 22 heures à une température voisine de 25°C, puis filtré sur verre fritté. La solution résultante est lavée successivement par 200 ml d'eau, 200 ml d'une solution aqueuse d'acide sulfurique à 5 %, 200 ml d'une solution aqueuse à 5% de bicarbonate de soude et 200 ml d'eau salée. Après concentration de la phase organique, on obtient une huile jaune qui cristallise rapidement. Le solide est broyé avec de l'eau distillée, puis séché sous pression réduite à 60°C. On obtient ainsi 25 g de l'ester (2-oxo-2-phényl)éthylique de l'acide (1-oxo-indane-4-yl)acétique sous forme de solide jaune fondant à 74°C.

### EXEMPLE 8

Une solution de 0,8 g d'acide (*E*)-3-(9-carboxyméthyl-4-oxo-5,10-dihydro-imidazo[1,2-a]indéno[1,2-e]pyrazin-2-yl)-acrylique dans 18 ml d'une solution aqueuse d'hydroxyde de sodium 0,32N est hydrogénée en présence de 0,04 g de charbon palladié à 10% pendant 12 heures à une température voisine de 40°C et sous une pression voisine de 5 bar. Le milieu réactionnel est filtré, puis acidifié par de l'acide chlorhydrique 1N jusqu'à pH 3. Le précipité apparu est lavé par 2x5 ml d'eau distillée, puis par 2x5 ml d'acétone et enfin séché sous vide (1 mm Hg; 0,13 kPa) à environ 60°C. On obtient 0,4 g d'acide 3-(9-carboxyméthyl-4-oxo-5,10-dihydro-imidazo[1,2-a]indéno[1,2-e]pyrazin-2-yl)-propionique sous forme de solide beige fondant au-dessus de 260°C. [Analyse C18H15N3O5, 1,3 H2O % calculé C : 61,19, H : 4,28, N : 11,89, O : 22,64, % trouvé C : 60,8, H : 4,4, N : 12,0, O : 22,1].

### EXEMPLE 9

On chauffe à reflux pendant 1 heure un mélange de 0,3 g de (*E*)-4-(2-éthoxycarbonylvinyl)-1-(4-éthoxycarbonylméthyl-1-oxo-indan-2-yl)imidazole-2-carboxylate d'éthyle, 7 ml d'acide acétique et 5 g d'acétate d'ammonium. Le mélange réactionnel est additionné de 7 ml d'eau distillée et l'insoluble est isolé par filtration, lavé par 2x5 ml d'eau distillée puis 2x5 ml d'acétone. Après séchage sous vide (1 mm Hg, 0,13 kPa) à environ 20°C, on obtient 0,15 g de (*E*)-3-(9-carboxyméthyl-4-oxo-5,10-dihydro-imidazo[1,2-a]indéno[1,2-e]pyrazin-2-yl)-acrylate d'éthyle sous forme d'un solide brun fondant au-dessus de 260°C, utilisé tel quel dans les synthèses ultérieures [RMN Spectre ¹H dans DMSO-d6, T=300K, δ en ppm (250 Mhz) : 1,22 (3H, t, J=6Hz, CH₃), 1,30 (3H, t, J=6Hz, CH₃), 3,87 (2H, s, CH₂), 4,00 (2H, s, CH₂), 4,15 (2H, q, J=6Hz, OCH₂), 4,25 (2H, q, J=6Hz, OCH₂), 6,70 (1H, d, J=16Hz, CH éthylénique), 7,28 (1H, d, J=7Hz, CH arom.), 7,42 (1H, t, J=7Hz, CH arom.), 7,70 (1H, d, J=16Hz, CH éthylénique), 7,84 (1H, d, J=7Hz, CH arom.), 8,40 (1H, s, CH imidazole), 12,00 (1H, s large, NHCO)].

On chauffe à reflux pendant 48 heures un mélange de 1,45 g de (*E*)-3-(9-carboxyméthyl-4-oxo-5,10-dihydro-imidazo[1,2-a]indéno[1,2-e]pyrazin-2-yl)-acrylate d'éthyle, 19 ml d'acide chlorhydrique concentré et 78 ml d'acide acétique. Le mélange réactionnel est filtré et le solide est lavé par 2x20 ml d'eau distillée, puis par 2x10 ml d'acétone et enfin séché sous vide (1 mmHg, 0,13 kPa) à environ 60°C. On obtient 0,94 g d'acide (*E*)-3-(9-carboxyméthyl-4-oxo-5,10-dihydro-imidazo[1,2-a]indéno[1,2-e]pyrazine-2-yl)-acrylique sous forme de solide beige fondant au-dessus de 260°C [Analyse C18H13N3O5, 0,89 HCI, 0,91 H2O % calculé C : 61,54, H : 3,73, N : 11,96, O : 22,77, % trouvé C : 61,8, H : 3,4, N : 11,9].

Le (*E*)-4-(2-éthoxycarbonylvinyl)-1-(7-éthoxycarbonylméthyl-3-oxo-indan-2-yl)imidazole-2-carboxylate d'éthyle peut être préparé de la manière suivante : sous couverture d'argon on porte à reflux un mélange de 0,3 g de (*E*)-4(5)-(2-éthoxycarbonylvinyl)-1*H*-imidazole-2-carboxylate d'éthyle, 6 ml d'acétone et 0,86 g de carbonate de potassium. On ajoute alors une solution de 0,37 g de (2-bromo-1-oxo-indan-4-yl)-acétate d'éthyle dans 4 ml d'acétone et l'on poursuit le reflux pendant 1 heure. Le milieu réactionnel est filtré et le filtrat est évaporé au rotavapor pour fournir 0,5 g d'une laque noire, que l'on purifie par chromatographie sur silice en éluant avec un mélange dichlorométhane-méthanol (99,5-0,5 en volumes). On obtient ainsi 0,2 g de (*E*)-4-(2-éthoxycarbonylvinyl)-1-(7-éthoxycarbonylméthyl-3-oxo-indan-2-yl)imidazole-2-carboxylate d'éthyle sous forme d'une meringue brune fondant à 53°C utilisé tel quel dans les synthèses ultérieures [RMN Spectre ¹H dans DMSO-d6, T=300K, δ en ppm (300 Mhz) : 1,18 (3H, t, J=6Hz, CH₃), 1,22 (3H, t, J=6Hz, CH₃), 1,30 (3H, t, J=6Hz, CH₃), 3,35 et 3,82 (1H chacun, respectivement dd, J=17 et 4Hz, et dd, J=6 et 17Hz, PhCH₂), 3,90 (2H, s, CH₂CO), 4,20 (6H, m, 3 OCH₂), 5,92 (1H, dd, J=6 et 4Hz, CHN), 6,55 (1H, d, J=16Hz, CH ethylénique), 7,58 (1H, t, J=7Hz, CH arom.), 7,60 (1H, d, J=16Hz, CH ethylénique), 7,75 (2H, d, J=7Hz, 2 CH arom.), 8,05 (1H, s, CH imidazole)].

### EXEMPLE 10

On chauffe à reflux pendant 3 heures un mélange de 0,6 g de 1-[7-(diéthoxyphosphorylméthyl)-3-oxo-indan-2-yl]imidazole-2,4-dicarboxylate de diéthyle, 18 ml d'acide acétique et 9,4 g d'acétate d'ammonium. Le mélange réactionnel est additionné de 20 ml d'eau distillée et l'insoluble est isolé par filtration, lavé par 2x5 ml d'eau distillée puis 2x5 ml d'acétone. Après séchage sous vide (1 mm Hg, 0,13 kPa) au voisinage de 20°C, on obtient 0,26 g de 9-(diéthoxyphosphorylméthyl)-4-oxo-5,10-dihydro-imidazo[1,2-a]indéno[1,2-e]pyrazine-2-carboxylate d'éthyle sous forme d'un solide brun fondant au-dessus de 260°C utilisé tel quel dans les synthèses ultérieures [RMN Spectre ¹H dans DMSO-d6, T=300K, δ en ppm (300 Mhz) : 1,15 (6H, t, J=6Hz, 2 CH₃), 1,35 (3H, t, J=6Hz, CH₃), 3,35 (2H, d, J=22Hz, PCH₂), 3,98 (4H, q, J=6Hz, 2 OCH₂), 4,10 (2H, s, CH₂), 4,35 (2H, q, J=6Hz, OCH₂), 7,25 (1H, d, J=7Hz, CH arom.), 7,40 (1H, t, J=7Hz, CH arom.), 7,80 (1H, d, J=7Hz, CH arom.), 8,65 (1H, s, CH imidazole), 12,50 (1H, s large, NHCO)].

On chauffe à reflux pendant 2 heures un mélange de 0,6 g de 9-(diéthoxyphosphorylméthyl)-4-oxo-5,10-dihydro-imidazo[1 ,2-a]indéno[1,2-e]pyrazine-2-carboxylate d'éthyle, 30 ml d'acide bromhydrique à 30% dans l'acide acétique et 10 ml d'eau distillée. Le mélange réactionnel est filtré et le solide est lavé par 3x20 ml d'eau distillée, puis par 2x10 ml d'acétone et enfin séché sous vide (1 mm Hg, 0,13 kPa) au voisinage de 40°C. On obtient 0,3 g d'acide 4-oxo-9-phosphonométhyl-5,10-dihydro-imidazo[1,2-a]indéno[1,2-e]pyrazine-2-carboxylique sous forme de solide beige fondant au-dessus de 260°C [Analyse C15H12N3O6P, 0,09 HBr, 0,38 HCI, 1,54 H2O % calculé C : 49,87, H : 3,3, N : 11,63, O : 26,57, P : 8.57 % trouvé C : 49,8, H : 3,4, N : 11,1]

Le 1-[7-(diéthoxyphosphorylméthyl)-3-oxo-indan-2-yl]imidazole-2,4-dicarboxylate d'éthyle peut être préparé de la manière suivante : sous couverture d'argon on porte à reflux un mélange de 0,3 g de 1*H*-imidazole-2,4(5)-dicarboxylate d'éthyle, 20 ml d'acétone et 0,59 g de carbonate de potassium. On ajoute alors une solution de 1,0 g de 2-bromo-1-oxo-indan-4-ylméthylphosphonate de diéthyle dans 10 ml d'acétone et l'on poursuit le reflux pendant 2 heures. Le milieu réactionnel est filtré et le filtrat est évaporé au rotavapor. Le résidu d'évaporation est purifié par chromatographie sur silice en éluant avec un mélange acétate d'éthyle-acétone (95-5 en volumes). On obtient ainsi 0,76 g de 1-[7-(diéthoxyphosphorylméthyl)-3-oxo-indan-2-yl]imidazole-2,4-dicarboxylate d'éthyle sous forme d'une huile jaune [RMN Spectre ¹H dans DMSO-d6, T=300K, δ en ppm (250 Mhz) : 1,20 (9H, m, 3 CH₃), 1,35 (3H, t, J=6Hz, CH₃), 3,40 (3H,m, HCH et PCH₂), entre 3,80 et 4,40 (9H, m, HCH et 4 OCH₂), 5,90 (1H, dd, J=5 et 8Hz, NCH), 7,55 (1H, t, J=7Hz, CH arom.), 7,70 (1H, m, CH arom.), 8,40 (1H, s, CH imidazole)].

Le 2-bromo-1-oxo-indan-4-ylméthylphosphonate de diéthyle peut être préparé de la manière suivante : on chauffe à une température voisine de 50°C un mélange de 0,37 g de 1-oxo-indan-4-ylméthylphosphonate de diéthyle et 0,5 g de monohydrate de perbromure de pyridinium dans 5 ml d'acétone, pendant une heure et trente minutes. Le milieu réactionnel est concentré au rotavapor. Le résidu d'évaporation est repris par 20 ml d'éther diéthylique, la phase organique est lavée par 2x10 ml d'eau distillée puis séchée sur sulfate de magnésium et concentrée au rotavapor. On obtient 0,46 g de 2-bromo-1-oxo-indan-4-ylméthylphosphonate de diéthyle sous forme d'une huile jaune [RMN Spectre ¹H dans DMSO-d6, T=300K, δ en ppm (250 Mhz) : 1,20 (6H, m, 2 CH₃), 3,40 et 3,90 (1H chacun, m, CH₂), 3,48 (2H, d, J=15Hz, PCH₂), 4,00 (4H, m, OCH₂), 5,10 (1H, dd, J=6 et 2Hz, CHBr), 7,50 (1H, t, J=7Hz, CH arom.), 7,70 (2H, d, J=7Hz, 2 CH arom.)].

Le 1-oxo-indan-4-ylméthylphosphonate de diéthyle peut être préparé de la manière suivante : on chauffe à reflux un mélange de 0,5 g de 4-(bromométhyl)indan-1-one, 0,9 g de phosphate de triéthyle et 5 ml de xylène pendant 5 heures. Le milieu réactionnel est concentré au rotavapor, puis le résidu est purifié par chromatographie sur silice en éluant avec un mélange acétate d'éthyle-éther de pétrole (85-15 en volumes). On obtient 0,4 g de (2-bromo-1-oxo-indan-4-yl)méthylphosphonate de diéthyle sous forme d'une huile incolore [RMN Spectre ¹H dans DMSO-d6, T=300K, δ en ppm (250 Mhz) : 1,20 (6H, t, J=6Hz, 2 CH₃), 2,70 (2H, m, CH₂CO), 3,20 (2H, m, CH₂), 3,38 (2H, d, J=22Hz, PCH₂), 4,00 (2H, q, J=6Hz, OCH₂), 7,45 (1H, t, J=7Hz, CH arom.), 7,60 (2H, m, 2 CH arom.)].

La 4-(bromométhyl)indan-1-one peut être préparée selon Agric. Biol. Chem. 42(7), 1365-73 (1978).

### EXEMPLE 11

A une solution de 2,9 g de 1-(4-carbéthoxyméthyl-1-oxo-indane-2-yl)-4-(4-carbéthoxyphényl)imidazole-2-carboxylate d'éthyle dans 25 ml d'acide acétique on ajoute 4,38 g d'acétate d'ammonium et le mélange est agité pendant 2 heures à l'ébullition puis de nouveau pendant 2 heures après addition de 1 g d'acétate d'ammonium. Après refroidissement à 60°C, l'insoluble est séparé par filtration, lavé 3 fois avec 45 ml au total d'acide acétique, 3 fois avec 90 ml au total d'éther éthylique et séché sous pression réduite (0,5 mm Hg; 0,07 kpa) à 60°C. On obtient ainsi 1,8 g de 2-(4-carbéthoxyphényl)-4-oxo-4,5-dihydro-1 OH-imidazo[1,2-a]indéno[1,2-e]pyrazine-9-acétate d'éthyle sous forme d'un solide beige se décomposant sans fondre au-dessus de 260°C [Spectre ¹H dans DMSO-d6, T=300K, δ en ppm (300 MHz): 1,20 (3H, t, J=6Hz, CH₃), 1,35 (3H, t, J=6Hz, CH₃), 3,85 (2H, s, CH₂CO), 4,00 (2H, s, CH₂), 4,12 (2H, q, J=6Hz, OCH₂), 4,35 (2H, q, J=6Hz, OCH₂), 7,25 (1H, d, J=7Hz, CH arom), 7,40 (1H, t, J=7Hz, CH arom), 7,85 (1H, d, J=7Hz, CH arom), 8,05 et 8,15 (2H chacun, d, J=7Hz, PhCO₂H.), 8,70 (1H, s, CH imidazole), 12,10 (1H, s, NHCO)].

Une suspension agitée de 1,7 g de 2-(4-carbéthoxyphényl)-4-oxo-4,5-dihydro-10H-imidazo[1,2-a]indéno[1,2-e]pyrazine-9-acétate d'éthyle dans 40 ml d'une solution aqueuse 6N d'acide chlorhydrique est maintenue à l'ébullition pendant 24 heures. Après refroidissement, l'insoluble est séparé par filtration, lavé 3 fois avec 60 ml au total d'eau distillée et séché sous pression réduite (0,5 mm Hg; 0,07 kPa) à 45°C. 1,4 g de produit ainsi obtenu ( sur 1,46 g obtenu au total) est dissous dans 15 ml d'une solution aqueuse 1N de soude et la solution est agitée pendant 16 heures à 20°C. Après addition de 10 ml d'eau distillée, la solution est acidifiée avec une solution aqueuse d'acide chlorhydrique 10N. L'insoluble apparu est séparé par filtration, lavé 3 fois avec 30 ml au total d'eau distillée, 3 fois avec 30 ml au total d'acétone et séché sous pression réduite ( 0,5 mm Hg; 0,07 kpa) à 60°C. On obtient ainsi 1,2 g de chlorhydrate de l'acide 2-(4-carboxyphényl)-4-oxo-4,5-dihydro-10H-imidazo[1,2-a]indéno[1,2-e]pyrazine-9-acétique sous forme d'un solide beige se décomposant sans fondre au-dessus de 260°C [Spectre ¹H dans DMSO-d6, T=300K, δ en ppm (300 MHz): 3,75 (2H, s, CH₂CO), 4,00 (2H, s, CH₂), 7,25 (1H, d, J=7Hz, CH arom), 7,40 (1H, t, J=7Hz, CH arom), 7,80 (1H, d, J=7Hz, CH arom), 8,05 et 8,15 (2H chacun, d, J=7Hz, PhCO₂Et), 8,70 (1H, s, CH imidazole), 12,50 (1H, s, NHCO)].

Le 1-(4-carbéthoxyméthyl-1-oxo-indane-2-yl)-4-(4-carbéthoxyphényl)imidazole-2-carboxylate d'éthyle peut être préparé de la manière suivante : à une solution de 1,87 g de 4(5)-(4-carbéthoxyphényl)-1H-imidazole-2-carboxylate d'éthyle dans 50 ml d'acétone on ajoute 4,48 g de carbonate de potassium. Le mélange agité est chauffé à l'ébullition puis on ajoute goutte à goutte en 5 minutes une solution de 2,07 g de (2- bromo-1-oxo-indane-4-yl)acétate d'éthyle dans 15 ml d'acétone. L'agitation à l'ébullition est poursuivie pendant 3 heures et, après refroidissement à 20°C, l'insoluble est éliminé par filtration et le filtat est concentré à sec sous pression réduite (15 mm Hg; 2 kPa) à 40°C. Le produit obtenu est chromatographié sur une colonne de gel de silice neutre en éluant avec de l'acétate d'éthyle. On obtient ainsi 2,9 g de 1-(4-carbéthoxyméthyl-1-oxo-indane-2-yl)-4-(4-carbéthoxyphényl) imidazole-2-carboxylate d'éthyle sous la forme d'une meringue brun clair (Rf = 0,8; chromatographie sur couche mince de gel de silice, éluant: acétate d'éthyle).

### EXEMPLE 12

Une suspension agitée de 1 g de 2-(3-cyanophényl)-4-oxo-4,5-dihydro-10H-imidazo[1,2-a]indéno[1,2-e]pyrazine-9-acétate d'éthyle dans 50 ml d'une solution aqueuse d'acide chlorhydrique concentrée est maintenue à l'ébullition pendant 48 heures. Après refroidissement, l'insoluble est séparé par filtration, lavé 3 fois avec 30 ml au total d'eau distillée et séché sous pression réduite (0,5 mm Hg; 0,07 kPa) à 60°C. On obtient ainsi 0,8 g de chlorhydrate de l'acide 2-(3-carboxyphényl)-4-oxo-4,5-dihydro-10H-imidazo[1,2-a]indéno[1,2-e]pyrazine-9-acétique sous forme d'une poudre beige se décomposant sans fondre au dessus de 260°C [Spectre ¹H dans DMSO-d6, T=300K, δ en ppm (300 MHz): 3,80 (2H, s, CH₂CO), 4,00 (2H, s, CH₂), 7,30 (1H, d, J=7Hz, CH arom), 7,40 (1H, t, J=6Hz, CH arom), 7,65 (1H, t, J=6Hz, CH arom), 7,88 (1H, d, J=7Hz, CH arom), 8,00 et 8,25 (1H chacun, d, J=6Hz, 2 CH arom), 8,62 et 8,88 (1H chacun, s, 2 CH arom), 12,80 (1H, s, NHCO)].

Le 2-(3-cyanophényl)-4-oxo-4,5-dihydro-1 OH-imidazo[1,2-a]indéno[1,2-e]pyrazine-9-acétate d'éthyle peut être obtenu de la manière suivante : à une solution de 4,8 g de 1-(4-carbéthoxyméthyl-1-oxo-indane-2-yl)-4-(3-cyanophényl)imidazole-2-carboxylate d'éthyle dans 50 ml d'acide acétique on ajoute 8,08 g d'acétate d'ammonium et le mélange est agité pendant 3 heures a l'ébullition puis de nouveau pendant 2 heures après addition de 2,4 g d'acétate d'ammonium. Après refroidissement à 20°C, l'insoluble est séparé par filtration, lavé 2 fois avec 20 ml au total d'acide acétique, 3 fois avec 60 ml au total d'éther éthylique et séché sous pression réduite (0,5 mm Hg; 0,07 kpa) à 50°C. On obtient ainsi 2,76 g de 2-(3-cyanophényl)-4-oxo-4,5-dihydro-10H-imidazo[1,2-a]indéno[1,2-elpyrazine-9-acétate d'éthyle sous forme d'une poudre grise se décomposant sans fondre au-dessus de 260°C [Spectre ¹H dans DMSO-d6, T=300K, δ en ppm (300 MHz): 1,20 (3H, t, J=6Hz, CH₃), 3,87 (2H, s, CH₂CO), 4,00 (2H, s, CH₂), 4,13 (2H, q, J=6Hz, OCH₂), 7,25 (1H, d, J=7Hz, CH arom), 7,40 et 7,70 (1H chacun, t, J=7Hz, 2 CH arom), 7,80 (2H, d, J=7Hz, 2 CH arom), 8,33 (1H, d, J=7Hz, CH arom), 8,42 (1H, s, CH arom), 8,72 (1H, s, CH imidazole)].

Le 1-(4-carbéthoxyméthyl-1-oxo-indane-2-yl)-4-(3-cyanophényl)imidazole-2-carboxylate d'éthyle peut être préparé de la manière suivante : à une solution de 3,32 g de 4(5)-(3-cyanophényl)-1H-imidazole-2-carboxylate d'éthyle dans 100 ml d'acétone on ajoute 8,97 g de carbonate de potassium. Le mélange agité est chauffé à l'ébullition puis on ajoute goutte à goutte en 5 minutes une solution de 4,15 g de (2- bromo-1-oxo-indane-4-yl)acétate d'éthyle dans 30 ml d'acétone. L'agitation à l'ébullition est poursuivie pendant 4 heures, l'insoluble est éliminé par filtration à chaud et le filtat est concentré à sec sous pression réduite (15 mm Hg; 2 kPa) à 40°C. Le produit obtenu est chromatographié sur une colonne de gel de silice neutre en éluant avec de l'acétate d'éthyle. On obtient ainsi 4,86 g de 1-(4-carbéthoxyméthyl-1-oxo-indane-2-yl)-4-(3-cyanophényl)imidazole-2-carboxylate d'éthyle sous la forme d'une meringue brune [Spectre ¹H dans DMSO-d6, T=300K, δ en ppm (300 MHz): 1,15 (6H, t, J=6Hz, 2 CH₃), 3,30 et 3,80 (1H chacun, m, CH₂), 3,85 (2H, s, CH₂CO), entre 4,05 et 4,30 (4H, m, 2 OCH₂), 5,95 (1H, m, NCH), entre 7,50 et 7,90 (5H, m, 5 CH arom), 8,20 (1H, d, J=6Hz, CH arom.), 8,25 (1H, s, CH arom), 8,30 (1 H, s, H imidazole)].

Le 4(5)-(3-cyanophényl)-1H-imidazole-2-carboxylate d'éthyle peut être obtenu de la manière suivante : à une solution de 3,45 g de thiooxamate d'éthyle dans 130 ml de chlorure de méthylène on ajoute goutte à goutte à 20°C en 10 minutes une solution de 7,4 g de tétrafluoborate de triéthyloxonium dans 25 ml de chlorure de méthylène. Le mélange est agité pendant 16 heures à une température voisine de 20°C puis concentré à sec sous pression réduite (15 mm Hg; 2 kPa) à 45°C. Le produit obtenu (8,9 g) est dissous dans 25 ml d'acide acétique et on ajoute 5,1 g de chlorhydrate de 3-glycylbenzonitrile puis 4,26 g d'acétate de sodium anhydre. Le mélange est agité pendant 3 heures 30 minutes à 95°C puis est concentré à sec sous pression réduite (15 mm Hg; 2 kPa) à 50°C. Le produit obtenu est mis en suspension 2 fois dans 100 ml au total de chlorure de méthylène et, après filtration, les filtats sont réunis et concentrés à sec sous pression réduite (15 mm hg; 2 kpa) à 50°C. Le produit obtenu est dissous dans 300 ml d'acétate d'éthyle bouillant et, après filtation à chaud de la solution, refroidissement et conservation pendant 1 heure à une température voisine de 5°C, les cristaux apparus sont séparés par filtration et séchés. On obtient ainsi 3,3 g de 4(5)-(3-cyanophényl)-1H-imidazole-2-carboxylate d'éthyle fondant à 176°C.

Le chlorhydrate de 3-glycylbenzonitrile peut être préparé selon la méthode décrite dans le brevet européen 52442.

### EXEMPLE 13

A une solution de 2,15 g de 1-(4-carbéthoxyméthyl-1-oxo-indane-2-yl)-4-(2-carbéthoxyphényl)imidazole-2-carboxylate d'éthyle dans 25 ml d'acide acétique on ajoute 3,07 g d'acétate d'ammonium et le mélange est agité pendant 4 heures 30 minutes à l'ébullition puis concentré à sec sous pression réduite (15 mm Hg; 2 kPa) à 50°C.. Le produit obtenu est dissous dans 100 mi de chlorure de méthylène et la solution est lavée 3 fois avec 150 ml au total d'eau distillée, séchée sur du sulfate de sodium anhydre et concentrée à sec sous pression réduite (15 mm Hg; 2 kPa) à 50°C. Le produit obtenu est lavé 4 fois avec 200 ml au total d'éther éthylique et séché sous pression réduite ( 0,5 mm Hg; 0,07 kpa) à 60°C. On obtient ainsi 1,4 g de 2-(2-carbéthoxyphényl)-4-oxo-4,5-dihydro-1 OH-imidazo[1,2-a]indéno[1,2-e]pyrazine-9-acétate d'éthyle sous forme d'une poudre grise se décomposant sans fondre au-dessus de 260°C [Spectre ¹H dans DMSO-d6, T=300K, δ en ppm (250 MHz): 1,15 (3H, t, J=6Hz, CH₃), 1,25 (3H, t, J=6Hz, CH₃), 3,87 (2H, s, CH₂CO), 4,00 (2H, s, CH₂), 4,15 (2H, q, J=6Hz, OCH₂), 4,27 (2H, q, J=6Hz, OCH₂), 7,25 (1H, d, J=7Hz, CH arom), 7,42 et 7,50 (1H chacun, t, J=7Hz, 2 CH arom), 7,65 (2H, m, 2 CH arom), 7,85 (2H, m, 2 CH arom), 8,30 (1H, s, CH imidazole), 12,50 (1H, s, NHCO)].

Une solution agitée de 1,3 g de 2-(2-carbéthoxyphényl)-4-oxo-4,5-dihydro-10H-imidazo[1,2-a]indéno[1,2-e]pyrazine-9-acétate d'éthyle dans 45 ml d'une solution aqueuse 12N d'acide chlorhydrique est maintenue à l'ébullition pendant 64 heures. Après refroidissement, l'insoluble est séparé par filtration, lavé 3 fois avec 45 ml au total d'eau distillée et séché sous pression réduite (0,5 mm Hg; 0,07 kPa) à 45°C. On obtient ainsi 0,9 g de chlorhydrate de l'acide 2-(2-carboxyphényl)-4-oxo-4,5-dihydro-1 OH-imidazo[1,2-a]indéno[1,2-e]pyrazine-9-acétique sous forme d'un solide gris foncé se décomposant sans fondre au-dessus de 260°C [Spectre ¹H dans DMSO-d6, T=300K, δ en ppm (250 Mhz): 3,75 (2H, s, CH₂), 4,05 (2H, s, CH₂), 7,25 (1H, d, J=7Hz, CH arom.), 7,40 (1H, t, J=7Hz, CH arom.), entre 7,50 et 8,10 (5H, m, 5 CH arom.), 8,45 (1H, s, CH imidazole), 13,00 (1H, s large, NHCO)].

Le 1-(4-carbéthoxyméthyl-1-oxo-indane-2-yl)-4-(2-carbéthoxyphényl)imidazole-2-carboxylate d'éthyle peut être préparé de la manière suivante : à une solution de 1,55 g de 4(5)-(2-carbéthoxyphényl)-1H-imidazole-2-carboxylate d'éthyle dans 50 ml d'acétone on ajoute 4,14 g de carbonate de potassium. Le mélange agité est chauffé à l'ébullition puis on ajoute goutte à goutte en 5 minutes une solution de 1,8 g de (2- bromo-1-oxo-indane-4-yl)acétate d'éthyle dans 15 ml d'acétone. L'agitation à l'ébullition est poursuivie pendant 3 heures 30 minutes et, après refroidissement à 20°C, l'insoluble est éliminé par filtration et le filtat est concentré à sec sous pression réduite (15 mm Hg; 2 kPa) à 40°C. Le produit obtenu est chromatographié sur une colonne de gel de silice neutre en éluant avec un mélange chlorure de méthylène-acétate d'éthyle (80-20 en volumes). On obtient ainsi 2,28 g de 1-(4-carbéthoxyméthyl-1-oxo-indane-2-yl)-4-(2-carbéthoxyphényl)imidazole-2-carboxylate d'éthyle sous forme d'une huile épaisse orange [Spectre ¹H dans DMSO-d6, T=300K, δ en ppm (300 MHz): 1,10 (9H, m, 3 CH₃), 3,30 et 3,75 (1H chacun, m, CH₂), 3,80 (2H, s, CH₂CO), entre 4,00 et 4,30 (6H, m, 3 OCH₂), 5,85 (1H, m, CH), entre 7,30 et 7,70 (7H, m, 7 CH arom), 7,85 (1H, s, H imidazole)].

### EXEMPLE 14

A une solution de 2,1 g de 2-(2,4-diéthoxycarbonyl-imidazol-1-yl)-4-benzènesulfonamidocarbonylméthyl-indan-1-one et de 55 ml d'acide acétique, on ajoute sous agitation 3 g d'acétate d'ammonium. Le milieu réactionnel est porté à l'ébullition pendant 3 heures puis refroidi et versé dans un mélande de 60g de glace et 60 ml d'eau distillée. La suspension ainsi obtenue est filtrée sur verre fritté, lavée par deux fois 20 ml d'eau, 10 ml d'acétone et séchée sous pression réduite à 53°C. On obtient ainsi 0,92 g de 9-benzènesulfonamidocarbonylméthyl-4-oxo-4,5-dihydro-10H-imidazo[1,2-a]-indéno[1,2-e]-pyrazine-2-carboxylate d'éthyle sous forme de solide gris fondant au dessus de 260°C (Analyse C₂₄H₂₀N₄O₆S₁ : % calculé C : 58,53, H : 4,09, N : 11,38, S : 6,54; % trouvé C : 58,3, H : 4,1, N : 11,5, S : 6,9).

A une suspension de 0,6 g de 9-benzènesulfonamidocarbonylméthyl-4-oxo-4,5-dihydro-10H-imidazo[1,2-a]-indéno[1,2-e]-pyrazine-2-carboxylate d'éthyle dans 60 ml de dioxanne et 1,8 ml d'eau distillée, on ajoute goutte à goutte 5 ml de soude aqueuse 1N. Le milieu réactionnel est maintenu sous agitation pendant 20 heures, filtré sur verre fritté et le solide ainsi obtenu est lavé par deux fois 10 ml de dioxanne, séché à l'air, puis dissout dans 20 ml d'eau distillée. A cette solution est ajouté 0,1 g de noir animal. Après filtration sur papier , le filtrat est acidifié à pH voisin de 3-4 avec 3,5 ml d'acide chlorhydrique 1N. Après avoir laissé reposer à une température voisine de 20°C, la suspension est filtrée sur verre fritté. Le solide ainsi obtenu est lavé par 3 fois 10 ml d'eau et 10 ml d'acétone puis séché sous pression réduite à 50°C. On obtient ainsi 0,28 g d'un solide beige clair. Le filtrat est concentré sous pression réduite à 48°C et permet d'obtenir 0,07 g d'un solide blanc. Ces deux solides sont mis en suspension dans 35 ml de dioxanne et 1 ml d'eau distillée, à laquelle sont ajoutés goutte à goutte en maintenant la température à 20°C, 2,5 ml de soude 1N. Après 18 heures d'agitation, le milieu réactionnel est filtré sur verre fritté et le solide résultant est lavé par 2 fois 10 mi de dioxanne, séché à l'air puis dissout dans 10 ml d'eau distillée. A cette solution est ajoutée 2,1 ml d'acide chlorhydrique 1N en maintenant la température à 20°C. Après avoir laissé agité pendant 3 heures, la suspension est filtrée sur verre fritté, lavée par 2 fois 5 ml d'eau distillée et séchée sous pression réduite à 50°C. On obtient ainsi 0,28 g d'acide 9-benzènesulfonamidocarbonylméthyl-4-oxo-4,5-dihydro-10H-imidazo[1,2-a]-indéno[1,2-e]-pyrazine-2-carboxylique sous forme d'un solide beige fondant au dessus de 260°C (Analyse C₂₂H₁₆N₄O₆S₁, 1,94 H₂O % calculé C : 56,90, H : 3,47, N : 12,06, S : 6,90; % trouvé C : 56,4, H : 2,8, N : 11,9, S : 6,6).

La 2-(2,4-diéthoxycarbonyl-imidazol-1-yl)-4-benzènesulfonamidocarbonylméthyl-indan-1-one peut être obtenue de la manière suivante : d'une part, à une suspension de 2,4 g de 1-[4-(carboxyméthyl)-1-oxo-indan-2-yl]-imidazole-2,4-dicarboxylate de diéthyle dans 25 ml de tétrahydrofuranne, on ajoute par portions 1,95 g de 1,1'-carbonyl-diimidazole et le milieu réactionnel est maintenu sous agitation à une température voisine de 20°C pendant 1 heure 30 minutes. On obtient ainsi une solution A. D'autre part, à une suspension refroidie à 0°C de 0,36 g d'hydrure de sodium à 80% et de 5 ml de tétrahydrofuranne sous atmosphère d'argon, on ajoute goutte à goutte une solution de 1,9 g de benzènesulfonamide dans 15 ml de tétrahydrofuranne. Le milieu réactionnel est alors ramené à une température voisine de 20°C et agité pendant 1 heure. Ce milieu est à nouveau refroidi à 0°C, et on ajoute alors goutte à goutte la solution A en maintenant la température à 0°C. Le milieu réactionnel est laissé sous agitation à cette température pendant 15 minutes puis ramené à une température voisine de 20°C et agité pendant 2 heures. Le milieu réactionnel est versé dans un mélange de 100 g de glace et 100 ml d'eau et repris par deux fois 100 ml de dichlorométhane. La phase aqueuse est acidifiée jusqu'à pH voisin de 4 avec 4 ml d'acide acétique. Cette phase est extraite par trois fois 100 ml de dichlorométhane. Les phases organiques réunies sont lavées par deux fois 100 ml d'eau distillée, séchées sur sulfate de magnésium, filtrées sur papier et concentrées sous pression réduite à 45°C. Le résidu solide ainsi obtenu est purifié par chromatographie sur silice avec de l'acétate d'éthyle comme éluant. On obtient ainsi 2,1 g de 2-(2,4-diéthoxycarbonyl-imidazol-1-yl)-4-benzènesulfonamido-carbonylméthyl-indan-1-one sous forme de solide crème utilisé tel quel pour la suite de la synthèse.

Le 1-[4-(carboxyméthyl)-1-oxo-indan-2-yl]-imidazole-2,4-dicarboxylate de diéthyle peut être obtenu de la manière suivante : à une solution de 75 ml de dioxanne chlorhydrique 6,5 N, on ajoute par portions 6,5 g de 1-[4-(tert-butoxycarbonylméthyl)-1-oxo-indan-2-yl]-imidazole-2,4-dicarboxylate de diéthyle et on maintient l'agitation pendant une heure à une température voisine de 20°C. Le milieu réactionnel est concentré sous pression réduite à 43°C et le résidu huileux ainsi obtenu est agité pendant 2 heures en présence de 150 ml d'éther diéthylique. La suspension ainsi obtenue est filtrée sur verre fritté, lavée par deux fois 25 ml d'éther diéthylique et séchée à l'air. On obtient ainsi 5,1 g de 1-[4-(carboxyméthyl)-1-oxo-indan-2-yl]-imidazole-2,4-dicarboxylate de diéthyle sous forme de solide crème fondant à 180°C.

Le 1 -[4-(tert-butoxycarbonylméthyl)-1-oxo-indan-2-yl]-imidazole-2,4-dicarboxylate de diéthyle peut être obtenu de la manière suivante : une suspension de 3,1 g d'imidazole-2,4-dicarboxylate de diéthyle, de 11 g de carbonate de potassium et de 90 ml d'acétone est portée sous agitation au reflux. On ajoute alors goutte à goutte en dix minutes, une solution de 5,2 g de (2-bromo-1-oxo-indane-4-yl)acétate de tertiobutyle dans 60 ml d'acétone. Après 4 heures d'agitation au reflux, le milieu réactionnel est refroidi et concentré sous pression réduite à 40°C. Le résidu solide brun ainsi obtenu est ensuite repris par 600 ml d'eau distillée et 600 ml de dichlorométhane. Après décantation, la phase aqueuse est reprise par deux fois 600 ml de dichlorométhane. Les phases organiques réunies sont lavées par 600 ml d'eau distillée, séchées sur sulfate de magnésium, filtrées sur papier et concentrées sous pression réduite à 35°C. On obtient ainsi 6,6 g de 1-[4-(tert-butoxycarbonylméthyl)-1-oxo-indan-2-yl]-imidazole-2,4-dicarboxylate de diéthyle sous forme de solide marron clair fondant à 175°C.

Le (2-bromo-1-oxo-indane-4-yl)acétate de tertiobutyle peut être obtenu de la manière suivante : une suspension de 9,6 g d'acide (2-bromo-1-oxo-indane-4-yl)acétique dans 175 ml de toluène est portée sous agitation à 80°C jusqu'à dissolution. A cette température, 31 ml de N,N-diméthylformamide-di-tert-butyl-acétal sont ajoutés goutte à goutte en 10 minutes et on maintient l'agitation pendant 15 minutes. Après refroidissement, le milieu réactionnel est versé sur 700 ml d'eau distillée et 480 ml d'acétate d'éthyle sont ajoutés. Après décantation, la phase organique est lavée par deux fois 300 ml de solution saturée en hydrogénocarbonate de sodium puis par deux fois 300 ml d'eau distillée. La phase organique est séchée sur sulfate de magnésium, filtrée sur papier et concentrée sous pression réduite à 40°C. Le résidu ainsi obtenu est purifié par chromatographie sur silice avec du dichlorométhane comme éluant. On obtient ainsi 4,2 g de (2-bromo-1-oxo-indane-4-yl)acétate de tertiobutyle sous forme d'un solide jaune fondant à 64°C.

L'acide (2-bromo-1-oxo-indane-4-yl)acétique peut être obtenu de la manière suivante : à une solution de 38 g d'acide (1-oxo-indane-4-yl)acétique dans 800 ml d'acide acétique sont ajoutés 4 ml d'acide bromhydrique à 47%. A cette solution refroidie à une température voisine de 15 °C, sont ajoutés goutte à goutte et en 10 minutes, 11 ml de brome puis le milieu réactionnel est maintenu sous agitation pendant une heure à une température voisine de 15°C. On laisse revenir le milieu réactionnel à une température voisine de 20°C et poursuit la réaction pendant 2 heures. Le milieu réactionnel est versé dans un mélange de 800 g de glace et 800 ml d'eau puis filtré sur verre fritté. Le filtrat est repris par trois fois 800 ml de dichlorométhane et les phases organiques réunies sont lavées par deux fois 800 ml d'eau distillée, séchées sur sulfate de magnésium, filtrées sur papier et concentrées sous pression réduite à 50°C. Le résidu solide ainsi obtenu est trituré dans 240 ml d'éther diisopropylique, filtré sur verre fritté et séché à l'air. On obtient ainsi 45,4 g d'acide (2-bromo-1-oxo-indane-4-yl)acétique sous forme de solide crème fondant à 120°C.

### EXEMPLE 15

Le 9-méthylsulfonamidocarbonylméthyl-4-oxo-4,5-dihydro-10H-imidazo[1,2-a]-indéno[1,2-e]-pyrazine-2-carboxylate d'éthyle peut être obtenu de la manière suivante : à une solution de 1,45 g de 2-(2,4-diéthoxycarbonyl-imidazol-1-yl)-4-méthylsulfonamidocarbonylméthyl-indan-1-one et de 50 ml d'acide acétique, on ajoute sous agitation 2,34 g d'acétate d'ammonium. Le milieu réactionnel est maintenu à l'ébullition pendant 4 heures puis refroidi et filtré sur verre fritté. Le résidu solide est lavée par deux fois 15 ml d'acide acétique, deux fois 25 ml d'eau, 25 ml d'acétone et séché sous pression réduite à 60°C. On obtient ainsi 0,8 g de 9-méthylsulfonamidocarbonylméthyl-4-oxo-4,5-dihydro-10H-imidazo[1 ,2-a]-indéno[1,2-e]-pyrazine-2-carboxylate d'éthyle sous forme de solide beige fondant au dessus de 260°C (R_{f} = 0,38 chromatographie sur couche mince de gel de silice, éluant : chloroforme/méthanol/ammoniaque : 12/6/1)].

L'acide 9-méthylsulfonamidocarbonylméthyl-4-oxo-4,5-dihydro-10H-imidazo[1,2-a]-indéno[1,2-e]-pyrazine-2-carboxylique peut être obtenu de la manière suivante : à une solution de 0,48 g de 9-méthylsulfonamidocarbonylméthyl-4-oxo-4,5-dihydro-10H-imidazo[1,2-al-indéno[1,2-e]-pyrazine -2-carboxylate d'éthyle, de 45 ml de dioxanne et de 1,3 ml d'eau distillée, on ajoute goutte à goutte 4,4 ml de soude aqueuse 1N. Le milieu réactionnel est maintenu sous agitation pendant 20 heures, filtré sur verre fritté et le solide ainsi obtenu est lavé par deux fois 20 ml de dioxanne, séché à l'air, puis sous pression réduite à 60°C. On obtient ainsi sous forme de sel disodique 0,46 g d'acide 9-méthylsulfonamidocarbonylméthyl-4-oxo-4,5-dihydro-10H-imidazo [1,2-a]-indéno[1,2-el-pyrazine-2-carboxylique sous forme d'un solide rose fondant au dessus de 260°C (Analyse C₁₇H₁₂N₄O₆S₁Na₂, 6,21 H₂O, 0,49 C₄H₈O₂ % calculé C : 45,75, H : 2,71, N : 12,55, S : 7,18; % trouvé C : 45,7, H : 2,3, N : 12,5, S: 7,2).

La 2-(2,4-diéthoxycarbonyl-imidazol-1-yl)-4-méthylsulfonamidocarbonylméthyl-indan-1-one peut être obtenue de la manière suivante : à une suspension de 2,6 g de 1-[4-(carboxyméthyl)-1-oxo-indan-2-yl]-imidazole-2,4-dicarboxylate de diéthyle dans 65 ml de tétrahydrofuranne, on ajoute par portions 1,61 g de 1,1'-carbonyl-diimidazole et le milieu réactionnel est maintenu sous agitation à une température voisine de 20°C pendant 30 minutes puis au reflux pendant 30 minutes. Après retour à une température voisine de 20°C, on ajoute au milieu réactionnel 0,61 g de méthylsulfonamide et dix minutes plus tard une solution de 0,98 g de 1,8-diazabicyclo[5.4.0]undec-7-ène dans 25 ml de tétrahydrofuranne. On maintient sous agitation pendant 18 heures puis le milieu réactionnel est versé dans 433 ml d'acide chlorhydrique 1N. A la solution obtenue, on ajoute 220 ml d'acétate d'éthyle et la phase aqueuse est extraite par 3 fois 110 ml d'acétate d'éthyle. Les phases organiques réunies sont lavées par 110 ml d'eau distillée, séchées sur sulfate de sodium, filtrées sur papier et concentrées sous pression réduite à 40°C. Le résidu solide ainsi obtenu est purifié par chromatographie sur silice avec un mélange dichlorométhane-méthanol (9-1 en volumes) comme éluant. On obtient ainsi 1,45 g de 2-(2,4-diéthoxycarbonyl-imidazol-1-yl)-4-méthylsulfonamidocarbonylméthyl-indan-1-one sous forme de meringue gris-rose fondant vers 139°C (pâteux).

Les médicaments selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention des conditions qui requièrent l'administration d'un antagoniste du récepteur AMPA ou d'un antagoniste du récepteur NMDA. Ces composés sont notamment utiles pour traiter ou prévenir toutes les ischémies et en particulier l'ischèmie cérébrale, les effets dus à une anoxie, l'évolution de maladies neurodégénératives, de la chorée d'HUNTINGTON, de la maladie d'ALZHEIMER et autres démences, de la sclérose latérale amyotrophique ou d'autres maladies du motoneurone, de l'atrophie olivo-pontocérébelleuse, de la maladie de PARKINSON, vis-à-vis des manifestations épileptogènes et/ou convulsives, les traumatismes cérébraux ou spinaux, les traumatismes liés à la dégénérescence de l'oreille interne ou de la rétine, du tinnitus, de l'anxiété, de la dépression, de la schizophrénie, du syndrome de TOURETTE, des encéphalopathies hépatiques, des troubles du sommeil, des désordres du déficit attentionnel, des troubles des conditions hormonales (excès de la sécrétion de HG ou HL, sécrétion de corticostérone), en tant qu'analgésiques, antiinflammatoires, antianorexiques, antimigraineux, antiémétiques et pour traiter les empoisonnements par des neurotoxines ou d'autres substances agonistes du récepteur NMDA ou AMPA, ainsi que les troubles neurologiques associés aux maladies virales telles que les méningites et encéphalites virales, le SIDA, la rage, la rougeole et le tétanos, pour la prévention, la tolérance et la dépendance des symptômes d'abstinence aux drogues, à l'alcool et de l'inhibition de l'accoutumance et de la dépendance aux opiacés, barbituriques, amphétamine et benzodiazépines, dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutiriqueaminoacidurie, l'encéphalopathie saturnine (intoxication chronique au plomb) et la déficience en sulfite oxydase.

Les doses dépendent de l'effet recherché, de la durée du traitement et de la voie d'administration utilisée; elles sont généralement comprises entre 10 mg et 100 mg par jour par voie orale pour un adulte avec des doses unitaires allant de 5 mg à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

## Revendications

1. Composés de formule : 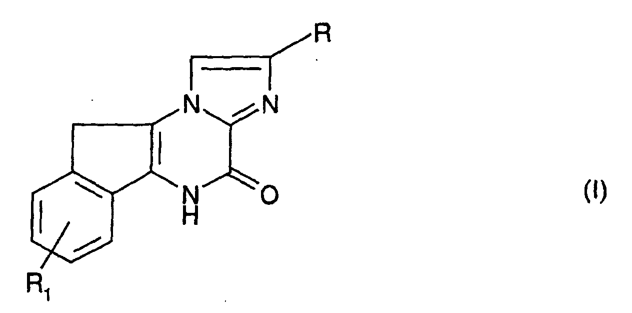 dans laquelle, soit R représente un atome d'hydrogène ou un radical -COOH, -alk-COOH, -PO₃H₂, -CH₂-PO₃H₂, -CH=CH-COOH ou phényle substitué par un radical carboxy,
R₁ représente un radical -alk-CN, -alk-COOH, -alk-Het, -alk-PO₃H₂ ou
-alk-CO-NH-SO₂R₂,
R₂ représente un radical alkyle ou phényle,
alk représente un radical alkyle,
Het représente un cycle tétrazole-5-yle
étant entendu que lorsque R représente un atome d'hydrogène ou un radical -COOH ou -PO₃H₂, R₁ ne peut pas représenter -alk-COOH
soit R représente un radical -COOH et R1 est en position 9 et représente un radical (4-phényl-1 H-imidazol-2-yl-méthyl),
et étant entendu que les radicaux alkyle contiennent 1 à 6 atomes de carbone en chaîne droite ou ramifiée,
les isomères E et Z des composés pour lesquels R représente un radical -CH=CH-COOH,
les racémiques, énantiomères et diastéréoisomères des composés pour lesquels R représente un radical -alk-COOH et/où R1 représente un radical -alk-CN, -alk-COOH, -alk-Het, -alk-PO₃H₂ ou -alk-CO-NH-SO₂R₂
et leurs sels.

2. Composés de formule (I) selon la revendication 1 pour lesquels le substituant R₁ est en position -8 ou -9.

3. Procédé de préparation des composés de formule (I) selon la revendication 1 pour lesquels R représente un atome d'hydrogène ou un radical -COOH, -alk-COOH, -PO₃H₂, -CH₂-PO₃H₂, -CH=CH-COOH ou phényle substitué par un radical carboxy, R₁ représente un radical -alk-CN,-alk-COOH, -alk-Het, -alk-PO₃H₂ ou -alk-CO-NH-SO₂R₂, R₂ représente un radical alkyle ou phényle **caractérisé en ce que** l'on cyclise, soit en présence d'acétate d'ammonium, soit en présence d'ammoniac, soit en présence d'acétate d'ammonium et d'ammoniac un dérivé de formule : 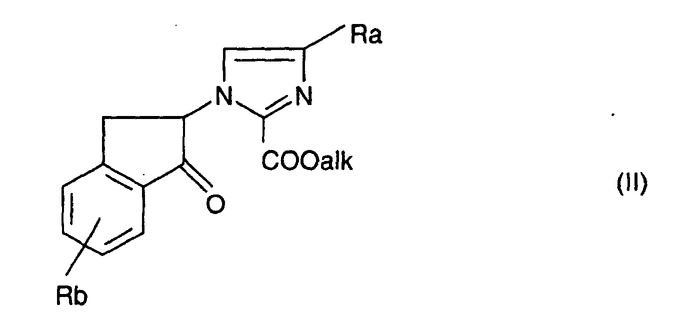 dans laquelle Ra représente un atome d'hydrogène ou un radical -COOalk, -alk-COOalk', -PO(Oalk)₂, -CH₂-PO(Oalk)₂, -CH=CH-COOalk' ou phényle substitué par un radical alcoxycarbonyle, Rb représente un radical -alk-CN, -alk-COOalk', -alk-PO(Oalk')₂, -alk-CO-NH-SO₂R₂ ou -alk-Het, alk et alk' représentent un radical alkyle, R₂ et Het ont les mêmes significations que dans la formule (I), puis hydrolyse, isole le produit et le transforme éventuellement en sel.

4. Procédé de préparation des composés de formule (I) selon la revendication 1 pour lesquels R₁ représente un radical -alk-Het **caractérisé en ce que** l'on cyclise, soit en présence d'acétate d'ammonium, soit en présence d'ammoniac, soit en présence d'acétate d'ammonium et d'ammoniac un dérivé de formule : 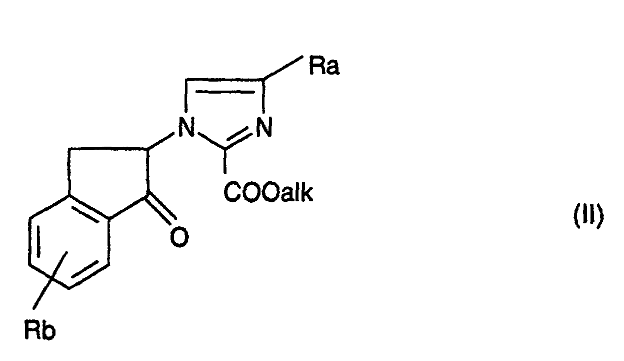 dans laquelle Ra représente un atome d'hydrogène ou un radical -COOalk, -alk-COOalk', -PO(Oalk)₂, -CH₂-PO(Oalk)₂, -CH=CH-COOalk ou phényle substitué par un radical alcoxycarbonyle, Rb représente un radical -alk-Het pour lequel Het représente un radical tétrazolyle-5-yle dont le tétrazole est substitué en position -1 ou -2 par un radical benzyle puis débenzyle et hydrolyse, isole le produit et le transforme éventuellement en sel.

5. Procédé de préparation des composés de formule (I) selon la revendication 1 pour lesquels R représente un radical -alk-COOH dans lequel alk est un radical alkyle contenant 2 atomes de carbone en chaîne droite **caractérisé en ce que** l'on hydrogène un composé de formule (I) correspondant pout lequel R représente un radical -CH=CH-COOH, isole le produit et le transforme éventuellement en sel.

6. Procédé de préparation des composés de formule (I) selon la revendication 1 pour lesquels R représente un radical phényle substitué par un radical carboxy **caractérisé en ce que** l'on hydrolyse un dérivé correspondant pour lequel le radical phényle est substitué par un radical cyano, isole le produit et le transforme éventuellement en sel.

7. Médicaments contenant en tant que principe actif au moins un composé de formule (I) selon la revendication 1.

8. Médicaments contenant en tant que principe actif au moins un composé de formule (I) selon la revendication 2.

9. Composés de formule : 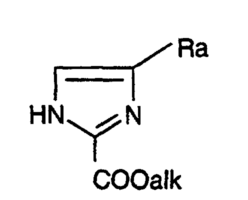 dans laquelle alk représente un radical alkyle et Ra représente un radical -alk-COOalk', -CH₂-PO(Oalk)₂, phényle substitué par un radical alcoxycarbonyle, -CH=CH-COOalk', ou -CH=CH-CHO dans lesquels alk et aik' représentent des radicaux alkyle de 1 à 6 atomes de carbone, en chaîne droite ou ramifiée.

10. Procédé de préparation des composés selon la revendication 9 pour lesquels Ra représente un radical -alk-COOalk', -CH₂-PO(Oalk)₂ ou phényle substitué par un radical alcoxycarbonyle **caractérisé en ce que** l'on fait réagir un dérivé H₂N-CH₂-CO-Rf sous forme d'un sel avec un acide minéral et dans lequel Rf représente un radical -alk-COOalk', -CH₂-PO(Oalk)₂ ou phényle substitué par un radical alcoxycarbonyle, sur un dérivé alkOOC-C(=NH)-Salk',BF₄H dans lequel alk et alk' représentent des radicaux alkyle et isole le produit.

11. Procédé de préparation des composés selon la revendication 9 pour lesquels Ra représente un radical -CH-CH-COOalk' **caractérisé en ce que** l'on fait réagir un alcool aliphatique (1-6C en chaîne droite ou ramifiée) en présence d'acide persulfurique sur un dérivé de formule: 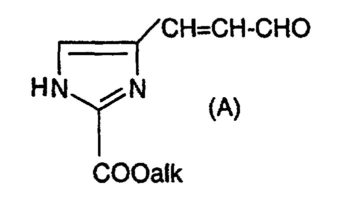 dans laquelle alk représente un radical alkyle puis isole le produit.

12. Procédé de préparation des composés selon la revendication 9 pour lesquels Ra représente un radical -alk(2C en chaîne droite)-COOalk' dans lequel alk' et alk représentent un radical alkyle **caractérisé en ce que** l'on hydrogène un dérivé de formule : 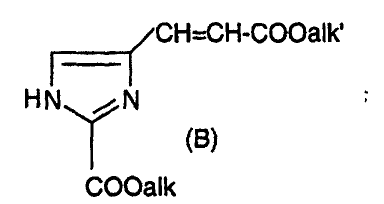 dans laquelle alk et alk' représentent un radical alkyle et isole le produit.

13. Procédé de préparation des composés selon la revendication 9 pour lesquels Ra représente un radical -CH=CH-CHO **caractérisé en ce que** l'on fait réagir un dérivé de formule : 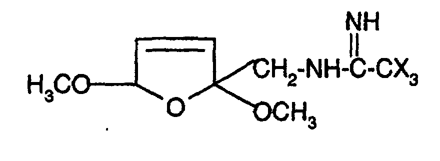 dans laquelle X représente un atome d'halogène ou un radical alcoxy avec un acide minéral ou un acide organique puis avec un alcool aliphatique (1-6C) et isole le produit.

## Patentansprüche

1. Verbindungen der Formel (I) 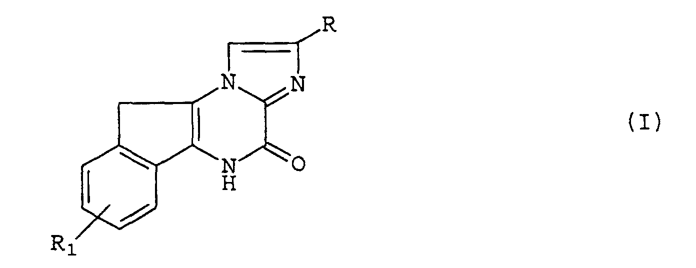 in der entweder
- R ein Wasserstoffatom oder einen Rest -COOH, -alk-COOH,
- PO₃H₂, -CH₂-PO₃H₂, -CH=CH-COOH oder Phenyl, substituiert durch einen Rest Carboxy, darstellt,
- R₁ einen Rest -alk-CN, -alk-COOH, -alk-Het, -alk-PO₃H₂ oder
- alk-CO-NH-SO₂R₂ darstellt,
- R₂ einen Rest Alkyl oder Phenyl bedeutet,
- alk ein Rest Alkyl ist,
- Het einen Ring Tetrazol-5-yl darstellt,
mit der Maßgabe, daß in dem Fall, wo R ein Wasserstoffatom oder einen Rest -COOH oder -PO₃H₂ darstellt, R₁ nicht -alk-COOH sein kann, oder
- R ein Rest -COOH ist und R₁ sich in Position 9 befindet und einen Rest (4-Phenyl-1H-imidazol-2-yl-methyl) darstellt,
mit der Maßgabe, daß die Reste Alkyl 1 bis 6 Kohlenstoffatome in gerader oder verzweigter Kette enthalten,
die Isomeren E und Z der Verbindungen, in denen R einen Rest -CH=CH-COOH darstellt,
die Racemate, Enantiomeren und Diastereoisomeren der Verbindungen, in denen R einen Rest -alk-COOH und/oder R₁ einen Rest -alk-CN, -alk-COOH, -alk-Het, -alk-PO₃H₂ oder -alk-CO-NH-SO₂R₂ darstellen, sowie ihre Salze.

2. Verbindungen der Formel (I) nach Anspruch 1, worin sich der Substituent R₁ in Position -8 oder -9 befindet.

3. Verfahren zur Herstellung der Verbindungen der Formel (I) nach Anspruch 1, in der R ein Wasserstoffatom oder einen Rest -COOH, -alk-COOH, -PO₃H₂, -CH₂-PO₃H₂, -CH=CH-COOH oder Phenyl, substituiert durch einen Rest Carboxy, darstellt, R₁ einen Rest -alk-CN, - alk-COOH, -alk-Het, -alk-PO₃H₂ oder -alk-CO-NH-SO₂R₂ darstellt, R₂ einen Rest Alkyl oder Phenyl bedeutet, **dadurch gekennzeichnet, daß** man ein Derivat der Formel (II) 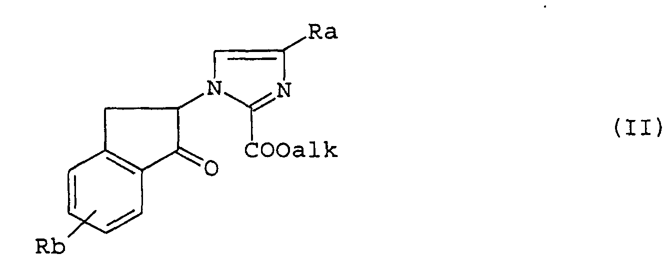 in der
Ra ein Wasserstoffatom oder einen Rest -COOalk, -alk-COOalk', -PO(Oalk)₂, -CH₂-PO(Oalk)₂, -CH=CH-COOalk' oder Phenyl, substituiert durch einen Rest Alkoxycarbonyl, darstellt, Rb einen Rest -alk-CN, -alk-COOalk', -alk-PO(Oalk')₂, -alk-CO-NH-SO₂R₂ oder -alk-Het darstellt, alk und alk' einen Rest Alkyl bedeuten, R₂ und Het die gleichen Bedeutungen wie in Formel (I) besitzen, entweder in Anwesenheit von Ammoniumacetat oder in Anwesenheit von Ammoniak oder in Anwesenheit von Ammoniumacetat und Ammoniak cyclisiert, anschließend hydrolysiert, das Produkt isoliert und gegebenenfalls in Salz überführt.

4. Verfahren zur Herstellung der Verbindungen der Formel (I) nach Anspruch 1, in der R₁ einen Rest -alk-Het darstellt, **dadurch gekennzeichnet, daß** man ein Derivat der Formel (II) 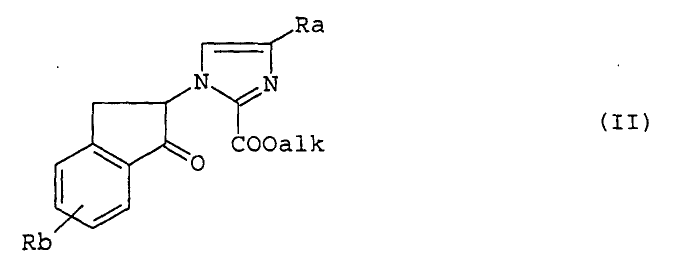 in der
Ra ein Wasserstoffatom oder einen Rest -COOalk, -alk-COOalk', -PO(Oalk)₂, -CH₂-PO(Oalk)₂, -CH=CH-COOalk oder Phenyl, substituiert durch einen Rest Alkoxycarbonyl, darstellt, Rb einen Rest -alk-Het darstellt, worin Het einen Rest Tetrazolyl-5-yl bedeutet, bei dem das Tetrazol in Position -1 oder -2 durch einen Rest Benzyl substituiert ist, entweder in Anwesenheit von Ammoniumacetat oder in Anwesenheit von Ammoniak oder in Anwesenheit von Ammoniumacetat und Ammoniak cyclisiert, anschließend debenzyliert und hydrolysiert, das Produkt isoliert und gegebenenfalls in Salz überführt.

5. Verfahren zur Herstellung der Verbindungen der Formel (I) nach Anspruch 1, in der R einen Rest -alk-COOH darstellt, worin alk ein Rest Alkyl mit 2 Kohlenstoffatomen in gerader Kette ist, **dadurch gekennzeichnet, daß** man eine entsprechende Verbindung der Formel (I), in der R einen Rest -CH=CH-COOH bedeutet, hydriert, das Produkt isoliert und gegebenenfalls in Salz überführt.

6. Verfahren zur Herstellung der Verbindungen der Formel (I) nach Anspruch 1, in der R einen Rest Phenyl, substituiert durch einen Rest Carboxy, darstellt, **dadurch gekennzeichnet, daß** man ein entsprechendes Derivat, worin der Rest Phenyl durch einen Rest Cyano substituiert ist, hydrolysiert, das Produkt isoliert und gegebenenfalls in Salz überführt.

7. Arzneimittel, enthaltend als Wirkstoff mindestens eine Verbindung der Formel (I) nach Anspruch 1.

8. Arzneimittel, enthaltend als Wirkstoff mindestens eine Verbindung der Formel (I) nach Anspruch 2.

9. Verbindungen der Formel 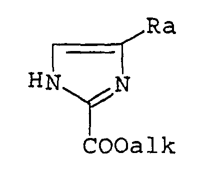 in der alk ein Rest Alkyl ist und Ra einen Rest -alk-COOalk', -CH₂-PO(Oalk)₂, Phenyl, substituiert durch einen Rest Alkoxycarbonyl, -CH=CH-COOalk' oder -CH=CH-CHO darstellt, worin alk und alk' Reste Alkyl mit 1 bis 6 Kohlenstoffatomen in gerader oder verzweigter Kette sind.

10. Verfahren zur Herstellung der Verbindungen nach Anspruch 9, worin Ra einen Rest -alk-COOalk', -CH₂-PO(Oalk)₂, oder Phenyl, substituiert durch einen Rest Alkoxycarbonyl, darstellt, **dadurch gekennzeichnet, daß** man ein Derivat H₂N-CH₂-CO-Rf in Form eines Salzes mit einer Mineralsäure und worin Rf einen Rest alk-COOalk', -CH₂-PO(Oalk)₂, oder Phenyl, substituiert durch einen Rest Alkoxycarbonyl, bedeutet, mit einem Derivat alkOOC-C(=NH)-Salk',BF₄H, worin alk und alk' Reste Alkyl sind, zur Reaktion bringt und das Produkt isoliert.

11. Verfahren zur Herstellung der Verbindungen nach Anspruch 9, worin Ra einen Rest -CH=CH-COOalk' darstellt, **dadurch gekennzeichnet, daß** man einen aliphatischen Alkohol (1 bis 6 Kohlenstoffatome in gerader oder verzweigter Kette) in Anwesenheit von Perschwefelsäure mit einem Derivat der Formel (A) 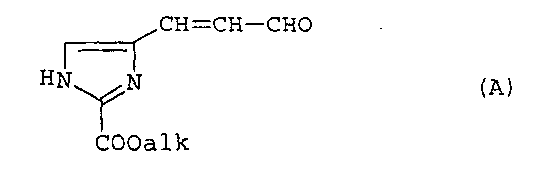 in der alk ein Rest Alkyl ist, zur Reaktion bringt und anschließend das Produkt isoliert.

12. Verfahren zur Herstellung der Verbindungen nach Anspruch 9, worin Ra einen Rest -alk(2C in gerader Kette)-COOalk' darstellt, worin alk und alk' Reste Alkyl sind, **dadurch gekennzeichnet, daß** man ein Derivat der Formel (B) 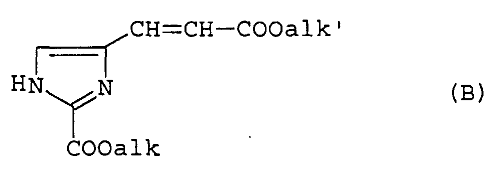 in der alk und alk' Reste Alkyl sind, hydriert und anschließend das Produkt isoliert.

13. Verfahren zur Herstellung der Verbindungen nach Anspruch 9, worin Ra einen Rest -CH=CH-CHO darstellt, **dadurch gekennzeichnet, daß** man ein Derivat der Formel 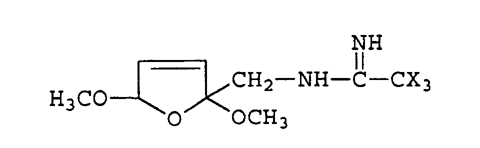 in der X ein Halogenatom oder einen Rest Alkoxy darstellt, mit einer Mineralsäure oder organischen Säure und danach mit einem aliphatischen Alkohol (1 bis 6 Kohlenstoffatome) zur Reaktion bringt und anschließend das Produkt isoliert.

## Claims

1. Compounds of formula: 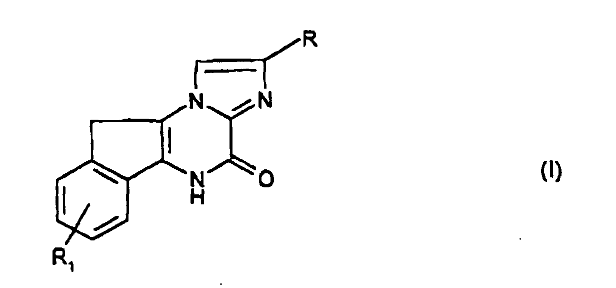 in which, either R represents a hydrogen atom or a -COOH radical, an -alk-COOH radical, a -PO₃H₂ radical, a -CH₂-PO₃H₂ radical, a -CH=CH-COOH radical or a phenyl radical substituted with a carboxyl radical,
R₁ represents an -alk-CN, -alk-COOH, -alk-Het,
-alk-PO₃H₂ or -alk-CO-NH-SO₂R₂ radical,
R₂ represents an alkyl or phenyl radical,
alk represents an alkyl radical,
Het represents a tetrazol-5-yl ring
it being understood that when R represent a hydrogen atom or a -COOH or -PO₃H₂ radical, R₁ cannot represent -alk-COOH
or R represents a -COOH radical and R₁ is at the 9-position and represents a (4-phenyl-lH-imidazol-2-ylmethyl) radical,
and it being understood that the alkyl radicals contain 1 to 6 carbon atoms in a straight or branched chain,
the E and Z isomers of the compounds for which R represents a -CH=CH-COOH radical,
the racemates, enantiomers and diastereoisomers of the compounds for which R represents an -alk-COOH radical and/or R₁ represents an -alk-CN, -alk-COOH, -alk-Het, -alk-PO₃H₂ or -alk-CO-NH-SO₂R₂ radical and their salts.

2. Compounds of formula (I) according to Claim 1, for which the substituent R₁ is at position 8 or 9.

3. Process for the preparation of the compounds of formula (I) according to Claim 1, for which R represents a hydrogen atom or a -COOH radical, an -alk-COOH radical, a -PO₃H₂ radical, a -CH₂-PO₃H₂ radical, a -CH=CH-COOH radical or a phenyl radical substituted with a carboxyl radical, R₁ represents an -alk-CN, -alk-COOH, -alk-Het, -alk-PO₃H₂ or -alk-CO-NH-SO₂R₂ radical, R₂ represents an alkyl or phenyl radical, **characterized in that** a derivative of formula: 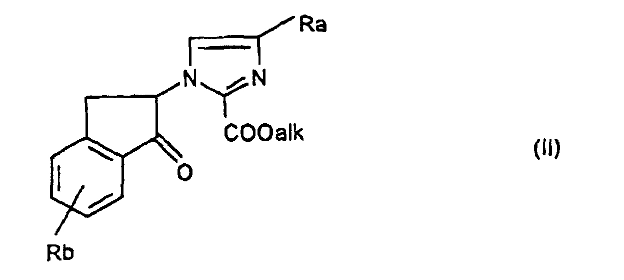 in which Ra represents a hydrogen atom or a -COOalk radical, an -alk-COOalk' radical, a -PO(Oalk)₂ radical, a -CH₂-PO(Oalk)₂ radical, a -CH=CH-COOalk' radical or a phenyl radical substituted with an alkoxycarbonyl radical, Rb represents an -alk-CN, -alk-COOalk', -alk-PO(Oalk')₂, -alk-CO-NH-SO₂R₂ or -alk-Het radical, alk and alk' represent an alkyl radical, R₂ and Het have the same meanings as in formula (I), is cyclized, either in the presence of ammonium acetate, or in the presence of ammonia, or in the presence of ammonium acetate and ammonia, then hydrolysed, and the product is isolated and optionally converted to a salt.

4. Process for the preparation of the compounds of formula (I) according to Claim 1, for which R₁ represents an -alk-Het radical, **characterized in that** a derivative of formula: 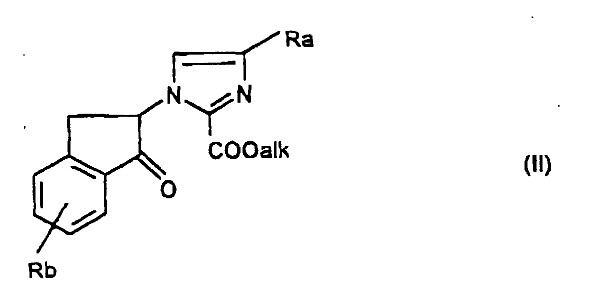 in which Ra represents a hydrogen atom or a -COOalk radical, an -alk-COOalk' radical, a -PO(Oalk)₂ radical, a -CH₂-PO(Oalk)₂ radical, a -CH=CH-COOalk radical or a phenyl radical substituted with an alkoxycarbonyl radical, Rb represents an -alk-Het radical for which Het represents a tetrazolyl-5-yl radical in which the tetrazole is substituted at position 1 or 2 with a benzyl radical, is cyclized, either in the presence of ammonium acetate, or in the presence of ammonia, or in the presence of ammonium acetate and ammonia, then debenzylated and hydrolysed, and the product is isolated and optionally converted to a salt.

5. Process for the preparation of the compounds of formula (I) according to Claim 1, for which R represents an -alk-COOH radical in which alk is an alkyl radical containing 2 carbon atoms in a straight chain, **characterized in that** a corresponding compound of formula (I) for which R represents a -CH=CH-COOH radical is hydrogenated, the product is isolated and optionally converted to a salt.

6. Process for the preparation of the compounds of formula (I) according to Claim 1 for which R represents a phenyl radical substituted with a carboxyl radical, **characterized in that** a corresponding derivative for which the phenyl radical is substituted with a cyano radical is hydrolysed, the product is isolated and optionally converted to a salt.

7. Medicaments containing, as active ingredient, at least one compound of formula (I) according to Claim 1.

8. Medicaments containing, as active ingredient, at least one compound of formula (I) according to Claim 2.

9. Compounds of formula: 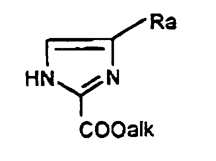 in which alk represents an alkyl radical and Ra represents an -alk-COOalk' radical, a -CH₂-PO(Oalk)₂ radical, a phenyl radical substituted with an alkoxycarbonyl radical, a -CH=CH-COOalk' radical, or a - CH=CH-CHO radical in which alk and alk' represent alkyl radicals of 1 to 6 carbon atoms in a straight or branched chain.

10. Process for the preparation of compounds according to Claim 9, for which Ra represents an -alk-COOalk' radical, a -CH₂-PO(Oalk)₂ radical or a phenyl radical substituted with an alkoxycarbonyl radical, **characterized in that** an H₂N-CH₂-CO-Rf derivative in the form of a salt with an inorganic acid and in which Rf represents an -alk-COOalk' radical, a -CH₂-PO(Oalk)₂ radical or a phenyl radical substituted with an alkoxycarbonyl radical, is reacted with an alkOOC-C(=NH)-Salk',BF₄H derivative in which alk and alk' represent alkyl radicals and the product is isolated.

11. Process for the preparation of the compounds according to Claim 9 for which Ra represents a -CH=CH-COOalk' radical, **characterized in that** an aliphatic alcohol (1-6C in a straight or branched chain) is reacted in the presence of persulphuric acid with a derivative of formula: 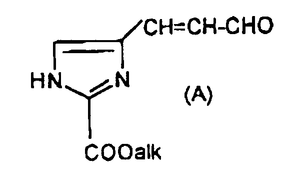 in which alk represents an alkyl radical and then the product is isolated.

12. Process for the preparation of the compounds according to Claim 9 for which Ra represents an -alk(2C in a straight chain)-COOalk' radical in which alk' and alk represent an alkyl radical, **characterized in that** a derivative of formula: 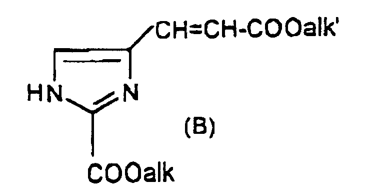 in which alk and alk' represent an alkyl radical, is hydrogenated and the product is isolated.

13. Process for the preparation of the compounds according to Claim 9 for which Ra represents a -CH=CH-CHO radical, **characterized in that** a derivative of formula: 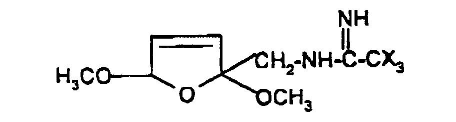 in which X represents a halogen atom or an alkoxy radical, is reacted with an inorganic acid or an organic acid and then with an aliphatic alcohol (1-6C) and the product is isolated.
